(19) Europäisches Patentamt
European Patent Office
Office européen des brevets

(11) EP 3 078 634 A1

(12) DEMANDE DE BREVET EUROPEEN

(43) Date de publication:
12.10.2016 Bulletin 2016/41

(51) Int Cl.:
C01B 33/193 (2006.01)
C08L 21/00 (2006.01)
C08K 3/36 (2006.01)
H01M 2/16 (2006.01)

(21) Numéro de dépôt: 16163156.9

(22) Date de dépôt: 13.08.2002

(84) Etats contractants désignés:
AT BE BG CH CY CZ DE DK EE ES FI FR GB GR IE IT LI LU MC NL PT SE SK TR

(30) Priorité: 13.08.2001 FR 0111001

(62) Numéro(s) de document de la (des) demande(s) initiale(s) en application de l'article 76 CBE:
02774872.2 / 1 419 106

(71) Demandeur: RHODIA CHIMIE
93300 Aubervilliers (FR)

(72) Inventeurs:
• VALERO, Rémi
01390 Saint-Jean de Thurigneux (FR)

• HERNANDEZ, Julien
92160 Antony (FR)

(74) Mandataire: Ferri, Isabella
Solvay S.A.
Viale Lombardia, 20
20021 Bollate (MI) (IT)

Remarques:
Cette demande a été déposée le 31-03-2016 comme demande divisionnaire de la demande mentionnée sous le code INID 62.

(54) NOUVEAU PROCÉDÉ DE PRÉPARATION DE SILICES, SILICES A DISTRIBUTION GRANULOMÉTRIQUE ET/OU RÉPARTITION POREUSE PARTICULIÈRES ET LEURS UTILISATIONS, NOTAMMENT POUR LE RENFORCEMENT DE POLYMÈRE

(57) L'invention concerne un nouveau procédé de préparation de silices, ainsi que des silices hautement structurées présentant les caractéristiques suivantes :
- une surface spécifique CTAB ($S_{CTAB}$) comprise entre 40 et 525 m²/g,
- une surface spécifique BET ($S_{BET}$) comprise entre 45 et 550 m²/g,
- une largeur Ld ((d84 - d16)/d50) de distribution de taille d'objets mesurée par granulométrie XDC après désagglomération aux ultra-sons d'au moins 0,91 et
- une répartition poreuse telle que le rapport $V_{(d5-d50)}/V_{(d5-d100)}$ est d'au moins 0,66.
Elle est également relative à l'utilisation de ces silices comme charges renforçantes de polymères.

EP 3 078 634 A1

**Description**

La présente invention concerne un nouveau procédé de préparation de silice, des silices à distribution granulométrique et/ou répartition poreuse particulières, se présentant notamment sous forme de poudre, de billes sensiblement sphériques ou de granulés, et leurs applications, telles que le renforcement des polymères.

Il est connu d'employer des charges blanches renforçantes dans les polymères, en particulier les élastomères, comme par exemple de la silice précipitée.

Le but de l'invention est de proposer notamment une charge alternative pour les compositions de polymères, aux caractéristiques atypiques, leur procurant de plus un compromis de propriétés très satisfaisant, en particulier au niveau de leurs propriétés mécaniques et dynamiques, sans pénaliser leurs propriétés rhéologiques.

L'invention propose d'abord un nouveau procédé de préparation de silice comprenant la réaction d'un silicate avec un agent acidifiant ce par quoi l'on obtient une suspension de silice, puis la séparation et le séchage de cette suspension, caractérisé en ce que la réaction du silicate avec l'agent acidifiant est réalisée selon les étapes successives suivantes:

(i) on forme un pied de cuve aqueux présentant un pH compris entre 2 et 5,
(ii) on ajoute audit pied de cuve, simultanément, du silicate et de l'agent acidifiant, de telle manière que le pH du milieu réactionnel soit maintenu entre 2 et 5,
(iii) on arrête l'addition de l'agent acidifiant tout en continuant l'addition de silicate dans le milieu réactionnel jusqu'à l'obtention d'une valeur du pH du milieu réactionnel comprise entre 7 et 10,
(iv) on ajoute au milieu réactionnel, simultanément, du silicate et de l'agent acidifiant, de telle manière que le pH du milieu réactionnel soit maintenu entre 7 et 10,
(v) on arrête l'addition du silicate tout en continuant l'addition de l'agent acidifiant dans le milieu réactionnel jusqu'à l'obtention d'une valeur du pH du milieu réactionnel inférieure à 6.

Il a été ainsi trouvé que la succession d'étapes particulières, et en particulier la présence d'une première addition simultanée d'agent acidifiant et de silicate en milieu acide à pH entre 2 et 5 et d'une seconde addition simultanée d'agent acifiant et de silicate en milieu basique à pH compris entre 7 et 10, constituaient des conditions importantes pour conférer aux produits obtenus leurs caractéristiques et propriétés particulières.

Le choix de l'agent acidifiant et du silicate se fait d'une manière bien connue en soi.

On utilise généralement comme agent acidifiant un acide minéral fort tel que l'acide sulfurique, l'acide nitrique ou l'acide chlorhydrique, ou un acide organique tel que l'acide acétique, l'acide formique ou l'acide carbonique.

L'agent acidifiant peut être dilué ou concentré ; sa normalité peut être comprise entre 0,4 et 36 N, par exemple entre 0,6 et 1,5 N.

En particulier, dans le cas où l'agent acidifiant est l'acide sulfurique, sa concentration peut être comprise entre 40 et 180 g/l, par exemple entre 60 et 130 g/l.

On peut par ailleurs utiliser en tant que silicate toute forme courante de silicates tels que métasilicates, disilicates et avantageusement un silicate de métal alcalin, notamment le silicate de sodium ou de potassium.

Le silicate peut présenter une concentration (exprimée en $SiO_2$) comprise entre 40 et 330 g/l, par exemple entre 60 et 300 g/l, en particulier entre 60 et 260 g/l.

De manière générale, on emploie, comme agent acidifiant, l'acide sulfurique, et, comme silicate, le silicate de sodium.

Dans le cas où l'on utilise le silicate de sodium, celui-ci présente, en général, un rapport pondéral $SiO_2/Na_2O$ compris entre 2,5 et 4, par exemple entre 3,2 et 3,8.

En ce qui concerne plus particulièrement le procédé de préparation de l'invention, la réaction du silicate avec l'agent acidifiant se fait d'une manière très spécifique selon les étapes suivantes.

On forme tout d'abord un pied de cuve aqueux présentant un pH compris entre 2 et 5.

De préférence, le pied de cuve formé présente un pH compris entre 2,5 et 5, notamment entre 3 et 4,5 ; ce pH est par exemple compris entre 3,5 et 4,5.

Ce pied de cuve initial peut être obtenu par ajout d'agent acidifiant à de l'eau de manière à obtenir une valeur de pH du pied de cuve entre 2 et 5, de préférence entre 2,5 et 5, notamment entre 3 et 4,5 et par exemple entre 3,5 et 4,5.

Il peut être également obtenu par ajout d'agent acidifiant à un mélange eau + silicate de manière à obtenir cette valeur de pH.

Il peut aussi être préparé par ajout d'agent acidifiant à un pied de cuve contenant des particules de silice préalablement formées à un pH inférieur à 7, de manière à obtenir une valeur de pH entre 2 et 5, de préférence entre 2,5 et 5, notamment entre 3 et 4,5 et par exemple entre 3,5 et 4,5.

Le pied de cuve formé dans l'étape (i) peut éventuellement comprendre un électrolyte. Néanmoins, de préférence, aucun électrolyte n'est ajouté au cours du procédé de préparation, en particulier dans l'étape (i).

Le terme d'électrolyte s'entend ici dans son acceptation normale, c'est-à-dire qu'il signifie toute substance ionique ou moléculaire qui, lorsqu'elle est en solution, se décompose ou se dissocie pour former des ions ou des particules chargées. On peut citer comme électrolyte un sel du groupe des sels des métaux alcalins et alcalino-terreux, notamment le sel du métal de silicate de départ et de l'agent acidifiant, par exemple le chlorure de sodium dans le cas de la réaction d'un silicate de sodium avec l'acide chlorhydrique ou, de préférence, le sulfate de sodium dans le cas de la réaction d'un silicate de sodium avec l'acide sulfurique.

La deuxième étape (étape (ii)) consiste en une addition simultanée d'agent acidifiant et de silicate, de telle manière (en particulier à des débits tels) que le pH du milieu réactionnel soit maintenu entre 2 et 5, de préférence entre 2,5 et 5, notamment entre 3 et 4,5, par exemple entre 3,5 et 4,5.

Cette addition simultanée est avantageusement réalisée de manière telle que la valeur du pH du milieu réactionnel soit constamment égale (à ± 0,2 près) à celle atteinte à l'issue de l'étape initiale (i).

Puis, dans une étape (iii), on arrête l'addition de l'agent acidifiant tout en continuant l'addition de silicate dans le milieu réactionnel de manière à obtenir une valeur du pH du milieu réactionnel comprise entre 7 et 10, de préférence entre 7,5 et 9,5.

Il peut alors être avantageux d'effectuer juste après cette étape (iii) et donc juste après l'arrêt de l'addition de silicate, un mûrissement du milieu réactionnel, notamment au pH obtenu à l'issue de l'étape (iii), et en général sous agitation ; ce mûrissement peut par exemple durer de 2 à 45 minutes, en particulier de 5 à 25 minutes et ne comporte préférentiellement ni addition d'agent acidifiant, ni addition de silicate.

Après l'étape (iii) et l'éventuel mûrissement, on procède à une nouvelle addition simultanée d'agent acidifiant et de silicate, de telle manière (en particulier à des débits tels) que le pH du milieu réactionnel soit maintenu entre 7 et 10, de préférence entre 7,5 et 9,5.

Cette seconde addition simultanée (étape (iv)) est avantageusement réalisée de manière telle que la valeur du pH du milieu réactionnel soit constamment égale (à ± 0,2 près) à celle atteinte à l'issue de l'étape précédente.

Il est à noter que l'on peut, entre l'étape (iii) et l'étape (iv), par exemple entre, d'une part, l'éventuel mûrissement suivant l'étape (iii), et, d'autre part, l'étape (iv), ajouter au milieu réactionnel de l'agent acidifiant, le pH du milieu réactionnel à l'issue de cette addition d'agent acidifiant étant cependant compris entre 7 et 9,5, de préférence entre 7,5 et 9,5.

Enfin, dans une étape (v), on arrête l'addition du silicate tout en continuant l'addition d'agent acidifiant dans le milieu réactionnel de manière à obtenir une valeur du pH du milieu réactionnel inférieure à 6, de préférence comprise entre 3 et 5,5, en particulier entre 3 et 5, par exemple entre 3 et 4,5.

Il peut alors être avantageux d'effectuer après cette étape (v) et donc juste après l'arrêt de l'addition d'agent acidifiant, un mûrissement du milieu réactionnel, notamment au pH obtenu à l'issue de l'étape (v), et en général sous agitation ; ce mûrissement peut par exemple durer de 2 à 45 minutes, en particulier de 5 à 20 minutes et ne comporte préférentiellement ni addition d'agent acidifiant, ni addition de silicate.

L'enceinte réactionnelle dans laquelle est mis en oeuvre l'ensemble de la réaction du silicate avec l'agent acidifiant est habituellement muni d'un équipement d'agitation et d'un équipement de chauffage adéquats.

L'ensemble de la réaction du silicate avec l'agent acidifiant est généralement réalisé entre 70 et 95 °C, en particulier entre 75 et 90 °C.

Selon une variante de l'invention, l'ensemble de la réaction du silicate avec l'agent acidifiant est effectué à une température constante, habituellement comprise entre 70 et 95 °C, en particulier entre 75 et 90 °C.

Selon une autre variante de l'invention, la température de fin de réaction est plus élevée que la témpérature de début de réaction : ainsi, on maintient la température au début de la réaction (par exemple au cours des étapes (i) à (iii)) de préférence entre 70 et 85 °C, puis on augmente la température, de préférence jusqu'à une valeur comprise entre 85 et 95 °C, valeur à laquelle elle est maintenue (par exemple au cours des étapes (iv) et (v)) jusqu'à la fin de la réaction.

On obtient, à l'issue des étapes qui viennent d'être décrites, une bouillie de silice qui est ensuite séparée (séparation liquide-solide).

La séparation mise en oeuvre dans le procédé de préparation selon l'invention comprend habituellement une filtration, suivie d'un lavage si nécessaire. La filtration s'effectue selon toute méthode convenable, par exemple au moyen d'un filtre presse, d'un filtre à bande, d'un filtre sous vide.

La suspension de silice ainsi récupérée (gâteau de filtration) est ensuite séchée.

Ce séchage peut se faire selon tout moyen connu en soi.

De préférence, le séchage se fait par atomisation. A cet effet, on peut utiliser tout type d'atomiseur convenable, notamment un atomiseur à turbines, à buses, à pression liquide ou à deux fluides. En général, lorsque la filtration est effectuée à l'aide d'un filtre presse, on utilise un atomiseur à buses, et, lorsque la filtration est effectuée à l'aide d'un filtre sous-vide, on utilise un atomiseur à turbines.

Il y a lieu de noter que le gâteau de filtration n'est pas toujours dans des conditions permettant une atomisation notamment à cause de sa viscosité élevée. D'une manière connue en soi, on soumet alors le gâteau à une opération de délitage. Cette opération peut être réalisée mécaniquement, par passage du gâteau dans un broyeur de type colloïdal ou à bille. Le délitage est généralement effectué en présence d'un composé de l'aluminium, en particulier d'aluminate de sodium et, éventuellement, en présence d'un agent acidifiant tel que décrit précédemment (dans ce dernier cas, le composé de l'aluminium et l'agent acidifiant sont généralement ajoutés de manière simultanée). L'opération de délitage permet notamment d'abaisser la viscosité de la suspension à sécher ultérieurement.

Lorsque le séchage est effectué à l'aide d'un atomiseur à buses, la silice susceptible d'être alors obtenue se présente habituellement sous forme de billes sensiblement sphériques.

A l'issue du séchage, on peut alors procéder à une étape de broyage sur le produit récupéré. La silice qui est alors susceptible d'être obtenue se présente généralement sous forme d'une poudre.

Lorsque le séchage est effectué à l'aide d'un atomiseur à turbines, la silice susceptible d'être alors obtenue peut se présenter sous la forme d'une poudre.

Enfin, le produit séché (notamment par un atomiseur à turbines) ou broyé tel qu'indiqué précédemment peut éventuellement être soumis à une étape d'agglomération, qui consiste par exemple en une compression directe, une granulation voie humide (c'est-à-dire avec utilisation d'un liant tel que eau, suspension de silice ...), une extrusion ou, de préférence, un compactage à sec. Lorsque l'on met en oeuvre cette dernière technique, il peut s'avérer opportun, avant de procéder au compactage, de désaérer (opération appelée également pré-densification ou dégazage) les produits pulvérulents de manière à éliminer l'air inclus dans ceux-ci et assurer un compactage plus régulier.

La silice susceptible d'être alors obtenue par cette étape d'agglomération se présente généralement sous la forme de granulés.

Les poudres, de même que les billes, de silice obtenues par le procédé selon l'invention offrent ainsi l'avantage, entre autre, d'accéder de manière simple, efficace et économique, à des granulés, notamment par des opérations classiques de mise en forme, telles que par exemple une granulation ou un compactage, sans que ces dernières n'entraînent de dégradations susceptibles de masquer, voire annihiler, les bonnes propriétés intrinsèques attachées à ces poudres ou ces billes, comme cela peut être le cas dans l'art antérieur en mettant en oeuvre des poudres classiques.

Le procédé de préparation selon l'invention permet notamment d'obtenir des silices, plutôt du type silices précipitées, qui, d'une part, sont hautement structurées et non friables, et, d'autre part, présentent généralement une bonne aptitude à la dispersion (dispersibilité) dans les polymères, confèrent à ceux-ci un compromis de propriétés très satisfaisant, en particulier au niveau de leurs propriétés dynamiques et mécaniques (notamment bon effet de renforcement et très bonne résistance à l'abrasion), sans pénaliser leurs propriétés rhéologiques. Les silices obtenues présentent de préférence une distribution granulométrique et/ou une répartition poreuse particulières.

Les silices susceptibles d'être obtenues par le procédé de l'invention constituent l'un des aspects de la présente invention.

D'autres objets de l'invention consistent en de nouvelles silices, plutôt du type silices précipitées, qui sont hautement structurées et qui possèdent une distribution granulométrique spécifique et/ou une répartition poreuse particulière ; en outre, elles présentent généralement une bonne aptitude à la dispersion (dispersibilité) dans les polymères, confèrent à ceux-ci un compromis de propriétés très satisfaisant, en particulier au niveau de leurs propriétés dynamiques (notamment diminution de la dissipation d'énergie en déformation (faible effet Payne), faibles pertes hystérétiques à haute température (notamment diminution de la tangente delta à 60 °C), sans pénaliser leurs propriétés rhéologiques (et donc sans pénaliser leur aptitude à la mise en oeuvre / mise en forme (par exemple, plus faible viscosité à cru à iso-surface spécifique)), et possèdent de bonnes propriétés mécaniques, en particulier un bon effet de renforcement, notamment en terme de modules, et une très bonne résistance à l'abrasion, d'où une résistance améliorée à l'usure pour les articles finis à base desdits polymères.

Dans l'exposé qui suit, la surface spécifique BET est déterminée selon la méthode de BRUNAUER - EMMET - TELLER décrite dans "The journal of the American Chemical Society", Vol. 60, page 309, février 1938 et correspondant à la norme internationale ISO 5794/1 (annexe D).

La surface spécifique CTAB est la surface externe déterminée selon la norme NF T 45007 (novembre 1987) (5.12).

La prise d'huile DOP est déterminée selon la norme NF T 30-022 (mars 1953) en mettant en oeuvre le dioctylphtalate.

Le pH est mesuré selon la norme ISO 787/9 (pH d'une suspension à 5 % dans l'eau).

La méthode d'analyse granulométrique XDC par sédimentation centrifuge, à l'aide de laquelle est mesuré, d'une part, les largeurs de distribution de taille d'objets de la silice, et, d'autre part, le mode XDC illustrant sa taille d'objets, est décrite ci-après :

*Matériel nécessaire*

- granulomètre à sédimentation centrifuge BI-XDC (BROOKHAVEN-INSTRUMENT X DISC CENTRIFUGE) commercialisé par la société Brookhaven Instrument Corporation)

- bécher forme haute de 50 ml
- éprouvette graduée de 50 ml
- sonde à ultra-sons BRANSON 1500 watts, sans embout, de diamètre de 13 mm,
- eau permutée
- cristallisoir rempli de glace
- agitateur magnétique

*Conditions de mesure*

- version DOS 1.35 du logiciel (fournie par le constructeur du granulomètre)
- mode fixe
- vitesse de rotation
- durée de l'analyse : 120 minutes
- densité (silice) : 2,1
- volume de la suspension à prélever : 15 ml

*Préparation de l'échantillon*

Ajouter dans le bécher forme haute 3,2 g de silice et 40 ml d'eau permutée.
Mettre le bécher contenant la suspension dans le cristallisoir rempli de glace.
Plonger la sonde à ultra-sons dans le bécher.
Désagglomérer la suspension pendant 16 minutes à l'aide de la sonde BRANSON de 1500 watts (utilisée à 60 % de la puissance maximale).
Lorsque la désagglomération est terminée, mettre le bécher sur un agitateur magnétique.

*Préparation du granulomètre*

Allumer l'appareil et laisser chauffer pendant 30 minutes.
Rincer 2 fois le disque à l'eau permutée.
Introduire dans le disque 15 ml de l'échantillon à analyser et mettre en agitation.
Entrer dans le logiciel les conditions de mesure mentionnées ci-dessus.
Faire les mesures.
Lorsque les mesures ont été effectuées :

Arrêter la rotation du disque.
Rincer plusieurs fois le disque à l'eau permutée.
Arrêter l'appareil.

*Résultats*

Dans le registre d'appareil, relever les valeurs des diamètres passant à 16 %, 50 % (ou médiane) et 84 % (% massique) ainsi que la valeur du Mode (la dérivée de la courbe granulométrique cumulée donne une courbe de fréquence dont l'abscisse du maximum (abscisse de la population principale) est appelé le Mode).
La largeur Ld de distribution de taille d'objets, mesurée par granulométrie XDC, après désagglomération aux ultra-sons (dans l'eau), correspond au rapport (d84 - d16)/d50 dans lequel dn est la taille pour laquelle on a n% de particules (en masse) de taille inférieure à cette taille (la largeur Ld de distribution est donc calculée sur la courbe granulométrique cumulée, prise dans sa totalité).
La largeur L'd de distribution de taille d'objets inférieure à 500 nm, mesurée par granulométrie XDC, après désagglomération aux ultra-sons (dans l'eau), correspond au rapport (d84 - d16)/d50 dans lequel dn est la taille pour laquelle on a n% de particules (en masse), par rapport aux particules de taille inférieure à 500 nm, de taille inférieure à cette taille (la largeur L'd de distribution est donc calculée sur la courbe granulométrique cumulée, tronquée au-dessus de 500 nm).
De plus, on peut mesurer, à l'aide de cette méthode d'analyse granulométrique XDC par sédimentation centrifuge, une taille moyenne (en masse) des particules (c'est-à-dire des particules secondaires ou agrégats), notée $d_W$, après dispersion, par désagglomération aux ultra-sons, de la silice dans l'eau. La méthode diffère de celle précédemment décrite sur le fait que la suspension formée (silice + eau permutée) est désagglomérée, d'une part, pendant 8 minutes, et, d'autre part, à l'aide d'une sonde à ultra-sons VIBRACELL 1,9 cm (commercialisée par la société Bioblock) de 1500 watts (utilisée à 60 % de la puissance maximale). Après analyse (sédimentation pendant 120 minutes) la distribution en masse des tailles de particules est calculée par le logiciel du granulomètre. La moyenne géométrique en masse des tailles de particules («geometric mean (Xg)» selon l'appellation du logiciel), notée $d_W$, est calculée par le logiciel à partir de l'équation suivante :

$$\log d_w = (\Sigma(i=1 \text{ à } i=n)\ m_i \log d_i) / \Sigma(i=1 \text{ à } i=n)\ m_i ,$$

$m_i$ étant la masse de l'ensemble des objets dans la classe de taille $d_i$.

Les volumes poreux donnés sont mesurés par porosimétrie au mercure ; la préparation de chaque échantillon se fait comme suit : chaque échantillon est préalablement séché pendant 2 heures en étuve à 200 °C, puis placé dans un récipient à essai dans les 5 minutes suivant sa sortie de l'étuve et dégazé sous vide, par exemple à l'aide d'une pompe à tiroirs rotatifs ; les diamètres de pores (porosimètre MI CROMERITICS Autopore III 9420) sont calculés par la relation de WASHBURN avec un angle de contact théta égal à 140° et une tension superficielle gamma égale à 484 Dynes/cm (ou N/m).

$V_{(d5 - d50)}$ représente le volume poreux constitué par les pores de diamètres compris entre d5 et d50, et $V_{(d5 - d100)}$ représente le volume poreux constitué par les pores de diamètres compris entre d5 et d100, dn étant ici le diamètre de pores pour lequel n% de la surface totale de tous les pores est apporté par les pores de diamètre supérieur à ce diamètre (la surface totale des pores ($S_0$) peut être déterminée à partir de la courbe d'intrusion de mercure).

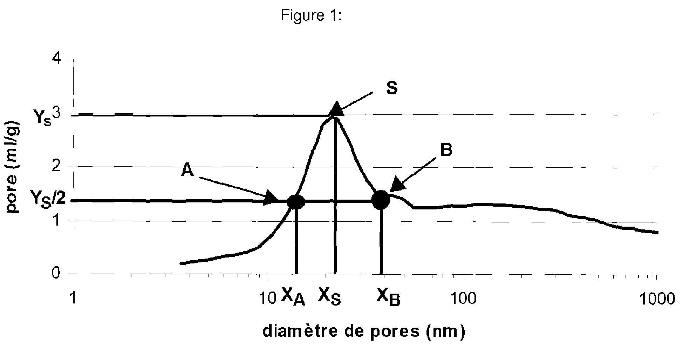

La largeur de distribution poreuse Idp s'obtient à partir de la courbe de répartition poreuse, comme indiqué à la figure 1, volume de pores (ml/g) en fonction du diamètre de pores (nm) : on relève les coordonnées du point S correspondant à la population principale, à savoir les valeurs du diamètre (nm) $X_S$ et du volume poreux (ml/g) $Y_S$. ; on trace une droite d'équation $Y = Y_S/2$. ; cette droite coupe la courbe de répartition poreuse en deux points A et B ayant pour abscisse (nm) respectivement $X_A$ et $X_B$ de part et d'autre de $X_S$ ; la largeur de distribution poreuse Idp est égale au rapport $(X_A - X_B) / X_S$.

Dans certains cas, l'aptitude à la dispersion (et à la désagglomération) des silices selon l'invention peut être quantifiée au moyen de tests spécifiques de désagglomération.

L'un des tests de désagglomération est réalisé selon le protocole suivant :

la cohésion des agglomérats est appréciée par une mesure granulométrique (par diffraction laser), effectuée sur une suspension de silice préalablement désagglomérée par ultra-sonification ; on mesure ainsi l'aptitude à la désagglomération de la silice (rupture des objets de 0,1 à quelques dizaines de microns). La désagglomération sous ultra-sons est effectuée à l'aide d'un sonificateur VIBRACELL BIOBLOCK (600 W), équipé d'une sonde de diamètre 19 mm. La mesure granulométrique est effectuée par diffraction laser sur un granulomètre SYMPATEC.

On pèse dans un pilulier (hauteur : 6 cm et diamètre : 4 cm) 2 grammes de silice et l'on complète à 50 grammes par ajout d'eau permutée : on réalise ainsi une suspension aqueuse à 4 % de silice qui est homogénéisée pendant 2 minutes par agitation magnétique. On procède ensuite à la désagglomération sous ultra-sons comme suit : la sonde étant immergée sur une longueur de 4 cm, on règle la puissance de sortie de manière à obtenir une déviation de l'aiguille du cadran de puissance indiquant 20 %. La désagglomération est effectuée pendant 420 secondes. On réalise ensuite la mesure granulométrique après avoir introduit dans la cuve du granulomètre un volume (exprimé en ml) connu de la suspension homogénéisée.

La valeur du diamètre médian $\varnothing_{50S}$ (ou diamètre médian Sympatec) que l'on obtient est d'autant plus faible que la silice présente une aptitude à la désagglomération élevée. On peut déterminer également le rapport (10 x volume de suspension introduite (en ml))/densité optique de la suspension détectée par le granulomètre (cette densité optique est de l'ordre de 20). Ce rapport est indicatif du taux de particules de taille inférieure à 0,1 μm qui ne sont pas détectées par le granulomètre. Ce rapport est appelé facteur de désagglomération (Sympatec) aux ultra-sons ($F_{DS}$).

Un autre test de désagglomération est réalisé selon le protocole suivant :

la cohésion des agglomérats est appréciée par une mesure granulométrique (par diffraction laser), effectuée sur une suspension de silice préalablement désagglomérée par ultra-sonification ; on mesure ainsi l'aptitude à la désagglomération de la silice (rupture des objets de 0,1 à quelques dizaines de microns). La désagglomération sous ultra-sons est effectuée à l'aide d'un sonificateur VIBRACELL BIOBLOCK (600 W), utilisé à 80 % de la puissance maximale, équipé d'une sonde de diamètre 19 mm. La mesure granulométrique est effectuée par diffraction laser sur un granulomètre MALVERN (Mastersizer 2000).

On pèse dans un pilulier (hauteur : 6 cm et diamètre : 4 cm) 1 gramme de silice et l'on complète à 50 grammes par ajout d'eau permutée : on réalise ainsi une suspension aqueuse à 2 % de silice qui est homogénéisée pendant 2 minutes par agitation magnétique. On procède ensuite à la désagglomération sous ultra-sons pendant 420 secondes. On réalise ensuite la mesure granulométrique après avoir introduit dans la cuve du granulomètre la totalité de la suspension homogénéisée.

La valeur du diamètre médian $\varnothing_{50M}$ (ou diamètre médian Malvern) que l'on obtient est d'autant plus faible que la silice présente une aptitude à la désagglomération élevée. On peut déterminer également le rapport (10 x valeur de l'obscuration du laser bleu) / valeur de l'obscuration du laser rouge. Ce rapport est indicatif du taux de particules de taille inférieure à 0,1 $\mu$m. Ce rapport est appelé facteur de désagglomération (Malvern) aux ultra-sons ($F_{DM}$).

Une vitesse de désagglomération, notée $\alpha$, peut être mesurée au moyen d'un autre test de désagglomération aux ultra-sons, à 100% de puissance d'une sonde de 600 watts, fonctionnant en mode pulsé (soit : 1 seconde ON, 1 seconde OFF) afin d'éviter un échauffement excessif de la sonde ultrasons pendant la mesure. Ce test connu, faisant notamment l'objet de la demande WO99/28376 (voir également les demandes WO99/28380, WO00/73372, WO00/73373), permet de mesurer en continu l'évolution de la taille moyenne (en volume) des agglomérats de particules durant une sonification, selon les indications ci-après. Le montage utilisé est constitué d'un granulomètre laser (type "MASTERSIZER S", commercialisé par Malvern Instruments - source laser He-Ne émettant dans le rouge, longueur d'onde 632,8 nm) et de son préparateur ("Malvern Small Sample Unit MSX1"), entre lesquels a été intercalée une cellule de traitement en flux continu (BIOBLOCK M72410) munie d'une sonde à ultra-sons (sonificateur 12,7 mm type VIBRACELL de 600 watts commercialisé par la société Bioblock). Une faible quantité (150 mg) de silice à analyser est introduite dans le préparateur avec 160 ml d'eau, la vitesse de circulation étant fixée à son maximum. Au moins trois mesures consécutives sont réalisées pour déterminer selon la méthode de calcul connue de Fraunhofer (matrice de calcul Malvern 3$$D) le diamètre initial moyen (en volume) des agglomérats, noté $d_V[0]$. La sonification (mode pulsé : 1 s ON, 1 s OFF) est ensuite établie à une puissance de 100 % (soit 100 % de la position maximum du "tip amplitude") et on suit durant 8 minutes environ l'évolution du diamètre moyen (en volume) $d_V[t]$ en fonction du temps "t" à raison d'une mesure toutes les 10 secondes environ. Après une période d'induction (environ 3-4 minutes), il est observé que l'inverse du diamètre moyen (en volume) $1/d_V[t]$ varie linéairement, ou de manière sensiblement linéaire, avec le temps "t" (régime stable de désagglomération). La vitesse de désagglomération $\alpha$ est calculée par régression linéaire de la courbe d'évolution de $1/d_V[t]$ en fonction du temps "t", dans la zone de régime stable de désagglomération (en général, entre 4 et 8 minutes environ) ; elle est exprimée en $\mu m^{-1}.mn^{-1}$.

La demande WO99/28376 précitée décrit en détail un dispositif de mesure utilisable pour la réalisation de ce test de désagglomération aux ultrasons. Ce dispositif consiste en un circuit fermé dans lequel peut circuler un flux d'agglomérats de particules en suspension dans un liquide. Ce dispositif comporte essentiellement un préparateur d'échantillon, un granulomètre laser et une cellule de traitement. Une mise à la pression atmosphérique, au niveau du préparateur d'échantillon et de la cellule de traitement elle-même, permet l'élimination en continu des bulles d'air qui se forment durant la sonification (action de la sonde ultrasons). Le préparateur d'échantillon ("Malvern Small Sample Unit MSX1") est destiné à recevoir l'échantillon de silice à tester (en suspension dans le liquide) et à le faire circuler à travers le circuit à la vitesse préréglée (potentiomètre - vitesse maximum d'environ 3 l/mn), sous la forme d'un flux de suspension liquide. Ce préparateur consiste simplement en une cuve de réception qui contient, et à travers laquelle circule, la suspension à analyser. Il est équipé d'un moteur d'agitation, à vitesse modulable, afin d'éviter une sédimentation des agglomérats de particules de la suspension ; une mini-pompe centrifuge est destinée à assurer la circulation de la suspension dans le circuit ; l'entrée du préparateur est reliée à l'air libre via une ouverture destinée à recevoir l'échantillon de charge à tester et/ou le liquide utilisé pour la suspension. Au préparateur est connecté un granulomètre laser ("Mastersizer S") dont la fonction est de mesurer en continu, à intervalles de temps réguliers, la taille moyenne en volume "$d_V$" des agglomérats, au passage du flux, grâce à une cellule de mesure à laquelle sont couplés les moyens d'enregistrement et de calcul automatiques du granulomètre. On rappelle ici brièvement que les granulomètres laser exploitent, de manière connue, le principe de la diffraction de la lumière par des objets solides mis en suspension dans un milieu dont l'indice de réfraction est différent de celui du solide. Selon la théorie de Fraunhofer, il existe une relation entre la taille de l'objet et l'angle de diffraction de la lumière (plus l'objet est petit et plus l'angle de diffraction sera élevé). Pratiquement, il suffit de mesurer la quantité de lumière diffractée pour différents angles de diffraction pour pouvoir déterminer la distribution de taille (en volume) de l'échantillon, $d_V$ correspondant à la taille moyenne en volume de cette distribution ($d_V = \sum(n_i d_i^4) / \sum(n_i d_i^3)$ avec ni nombre d'objets de la classe de taille ou diamètre $d_i$). Intercalée entre le préparateur et le granulomètre laser se trouve enfin une cellule de traitement équipée d'une sonde ultrasons, pouvant fonctionner en mode continu ou pulsé, destinée à casser en continu les agglomérats de particules au passage du flux. Ce flux est thermostaté par l'intermédiaire d'un circuit de refroidissement disposé, au niveau de la cellule, dans une double enveloppe entourant la sonde, la température étant contrôlée par exemple par une sonde de température plongeant dans le liquide au niveau du préparateur.

Le nombre de Sears est déterminé selon la méthode décrite par G. W. SEARS dans un article de «Analytical Chemistry, vol. 28, No. 12, décembre 1956 », intitulé «Determination of specific surface area of colloidal silica by titration with sodium hydroxyde ».

Le nombre de Sears est le volume de solution d'hydroxyde de sodium 0,1 M nécessaire pour élever le pH de 4 à 9 d'une suspension de silice à 10 g/l en milieu chlorure de sodium à 200 g/l.

Pour cela, on prépare, à partir de 400 grammes de chlorure de sodium, une solution de chlorure de sodium à 200 g/l acidifiée à pH 3 avec une solution d'acide chlorhydrique 1 M. Les pesées sont effectuées à l'aide d'une balance de précision METTLER. On ajoute délicatement 150 ml de cette solution de chlorure de sodium dans un bécher de 250 ml dans lequel a été préalablement introduit une masse M (en g) de l'échantillon à analyser correspondant à 1,5 gramme de silice sèche. On applique à la dispersion obtenue des ultra-sons pendant 8 minutes (sonde à ultra-sons BRANSON de 1500 W, amplitude de 60 %, diamètre de 13 mm), le bécher étant dans un cristallisoir rempli de glace. Puis on homogénéise la solution obtenue par agitation magnétique, à l'aide d'un barreau aimanté de dimensions 25 mm x 5 mm. On vérifie que le pH de la suspension est inférieur à 4, en l'ajustant si nécessaire avec une solution d'acide chlorhydrique 1 M. On ajoute ensuite, à l'aide d'un pHmètre titreur Metrohm (titroprocesseur 672, dosimat 655), préalablement étalonné à l'aide de solutions tampon pH 7 et pH 4, une solution d'hydroxyde de sodium 0,1 M à un débit de 2 ml/min. (Le pHmètre titreur a été programmé comme suit : 1) Appeler le programme «Get pH», 2) Introduire les paramètres suivants : pause (temps d'attente avant le début du titrage) : 3 s, débit de réactif : 2 ml/min, anticipation (adaptation de la vitesse de titrage à la pente de la courbe de pH) : 30, stop pH : 9,40, EP critique (sensibilité de détection du point d'équivalence) : 3, report (paramètres d'impression du rapport de titrage) : 2,3,5 (c'est-à-dire création d'un rapport détaillé, liste de points de mesure, courbe de titrage)). On détermine par interpolation les volumes $V_1$ et $V_2$ exacts de la solution d'hydroxyde de sodium ajoutée pour obtenir respectivement un pH de 4 et un pH de 9. Le nombre de Sears pour 1,5 gramme de silice sèche est égal à $((V_2 - V_1) \times 150) / (ES \times M)$, avec :

$V_1$ : volume de solution d'hydroxyde de sodium 0,1 M à $pH_1 = 4$
$V_2$ : volume de solution d'hydroxyde de sodium 0,1 M à $pH_2 = 9$
M : masse de l'échantillon (g)
ES : extrait sec en %

La largeur de distribution de taille de pore peut éventuellement être également illustrée par le paramètre L/IF déterminé par porosimétrie au mercure. La mesure est réalisée à l'aide des porosimètres PASCAL 140 et PASCAL 440 commercialisés par ThermoFinnigan, en opérant de la façon suivante : une quantité d'échantillon comprise entre 50 et 500 mg (dans le cas présent 140 mg) est introduite dans une cellule de mesure. Cette cellule de mesure est installée sur le poste de mesure de l'appareil PASCAL 140. L'échantillon est ensuite dégazé sous vide, pendant le temps nécessaire pour atteindre une pression de 0,01 kPa (typiquement de l'ordre de 10 minutes). La cellule de mesure est ensuite remplie de mercure. La première partie (pressions inférieures à 400 kPa) de la courbe d'intrusion du mercure Vp = f(P), où Vp est le volume d'intrusion du mercure et P la pression appliquée, est déterminée sur le porosimètre PASCAL 140. La cellule de mesure est ensuite installée sur le poste de mesure du porosimètre PASCAL 440, la seconde partie de la courbe d'intrusion du mercure Vp = f(P) (pressions comprises entre 100 kPa et 400 MPa) étant déterminée sur le porosimètre PASCAL 440. Les porosimètres sont utilisés en mode «PASCAL», de façon à ajuster en permanence la vitesse d'intrusion du mercure en fonction des variations du volume d'intrusion. Le paramètre de vitesse en mode «PASCAL » est fixé à 5. Les rayons de pore Rp sont calculés à partir des valeurs de pression P à l'aide de la relation de WASHBURN, avec une hypothèse de pores cylindriques, en choisissant un angle de contact théta égal à 140° et une tension superficielle gamma égale à 480 Dynes/cm (ou N/m). Les volumes poreux Vp sont rapportés à la masse de silice introduite et exprimés en $cm^3/g$. Le signal Vp = f(Rp) est lissé en combinant un filtre logarithmique (paramètre de filtre «smooth dumping factor » F = 0,96) et un filtre à moyenne mobile (paramètre de filtre «number of points to average » f = 20). La distribution de taille de pore est obtenue en calculant la dérivée dVp/dRp de la courbe d'intrusion lissée. L'indice de finesse IF est la valeur de rayon de pore (exprimée en angströms) correspondant au maximum de la distribution de taille de pore dVp/dRp. On note L la largeur à mi-hauteur de la distribution de taille de pore dVp/dRp.

Le nombre de silanols par $nm^2$ de surface est déterminé par greffage de méthanol sur la surface de la silice. Dans un premier temps, 1 gramme de silice brute est mis en suspension dans 10 ml de méthanol, dans un autoclave de 110 ml (Top Industrie, référence 09990009). Un barreau aimanté est introduit et l'autoclave, fermé hermétiquement et calorifugé, est chauffé à 200 °C (40 bars) sur agitateur magnétique chauffant pendant 4 heures. L'autoclave est ensuite refroidi dans un bain d'eau froide. La silice greffée est récupérée par décantation et le méthanol résiduel est évaporé sous courant d'azote. Enfin, la silice greffée est séchée à 130 °C sous vide pendant 12 heures. La teneur en carbone est déterminée par analyseur élémentaire (analyseur NCS 2500 de CE Instruments) sur la silice brute et sur la silice greffée. Ce dosage sur la silice greffée est effectué dans les trois jours qui suivent la fin du séchage, l'humidité de l'air ou la chaleur pouvant en effet provoquer une hydrolyse du greffage méthanol. Le nombre de silanols par $nm^2$ est alors calculé par la formule suivante :

$$N_{SiOH/nm2} = [(\%C_g - \%C_b) \times 6{,}023 \times 10^{23}] / [S_{BET} \times 10^{18} \times 12 \times 100]$$

avec

$\%C_g$ : pourcentage massique de carbone présent sur la silice greffée
$\%C_b$ : pourcentage massique de carbone présent sur la silice brute
$S_{BET}$ : surface spécifique BET de la silice ($m^2$/g)

Il est maintenant proposé, selon une première variante de l'invention, une nouvelle silice caractérisée en ce qu'elle possède :

- une surface spécifique CTAB ($S_{CTAB}$) comprise entre 40 et 525 $m^2$/g,
- une surface spécifique BET ($S_{BET}$) comprise entre 45 et 550 $m^2$/g,
- une largeur Ld ((d84 - d16)/d50) de distribution de taille d'objets mesurée par granulométrie XDC après désagglomération aux ultra-sons d'au moins 0,91, en particulier d'au moins 0,94, et
- une répartition du volume poreux en fonction de la taille des pores telle que le rapport $V_{(d5 - d50)}/V_{(d5 - d100)}$ est d'au moins 0,66, en particulier d'au moins 0,68.

La silice selon cette variante de l'invention possède par exemple :

- une largeur Ld ((d84 - d16)/d50) de distribution de taille d'objets mesurée par granulométrie XDC après désagglomération aux ultra-sons d'au moins 1,04 et
- une répartition du volume poreux en fonction de la taille des pores telle que le rapport $V_{(d5 - d50)}/V_{(d5 - d100)}$ est d'au moins 0,71.

Cette silice peut présenter un rapport $V_{(d5 - d50)}/V_{(d5 - d100)}$ d'au moins 0,73, en particulier d'au moins 0,74. Ce rapport peut être d'au moins 0,78, notamment d'au moins 0,80, voire d'au moins 0,84.

Une deuxième variante de l'invention consiste en une nouvelle silice caractérisée en ce qu'elle possède :

- une surface spécifique CTAB ($S_{CTAB}$) comprise entre 40 et 525 $m^2$/g,
- une surface spécifique BET ($S_{BET}$) comprise entre 45 et 550 $m^2$/g,
- une largeur de distribution poreuse Idp supérieure à 0,70, en particulier supérieure à 0,80, notamment supérieure à 0,85.

Cette silice peut présenter une largeur de distribution poreuse Idp supérieure à 1,05, par exemple à 1,25, voire à 1,40.

La silice selon cette variante de l'invention possède de préférence une largeur Ld ((d84 - d16)/d50) de distribution de taille d'objets mesurée par granulométrie XDC après désagglomération aux ultra-sons d'au moins 0,91, en particulier d'au moins 0,94, par exemple d'au moins 1,0.

Il est également proposé, selon une troisième variante de l'invention, une nouvelle silice caractérisée en ce qu'elle possède :

- une surface spécifique CTAB ($S_{CTAB}$) comprise entre 40 et 525 $m^2$/g,
- une surface spécifique BET ($S_{BET}$) comprise entre 45 et 550 $m^2$/g,
- une largeur L'd ((d84 - d16)/d50) de distribution de taille d'objets inférieure à 500 nm, mesurée par granulométrie XDC après désagglomération aux ultra-sons, d'au moins 0,95 et
- une répartition du volume poreux en fonction de la taille des pores telle que le rapport $V_{(d5 - d50)}/V_{(d5 - d100)}$ est d'au moins 0,71.

Cette silice peut présenter un rapport $V_{(d5 - d50)}/V_{(d5 - d100)}$ d'au moins 0,73, en particulier d'au moins 0,74. Ce rapport peut être d'au moins 0,78, notamment d'au moins 0,80, voire d'au moins 0,84.

Une quatrième variante de l'invention consiste en une nouvelle silice caractérisée en ce qu'elle possède :

- une surface spécifique CTAB ($S_{CTAB}$) comprise entre 40 et 525 $m^2$/g,
- une surface spécifique BET ($S_{BET}$) comprise entre 45 et 550 $m^2$/g,
- une largeur L'd ((d84 - d16)/d50) de distribution de taille d'objets inférieure à 500 nm, mesurée par granulométrie XDC après désagglomération aux ultra-sons, d'au moins 0,90, en particulier d'au moins 0,92, et

- une répartition du volume poreux en fonction de la taille des pores telle que le rapport $V_{(d5 - d50)}/V_{(d5 - d100)}$ est d'au moins 0,74.

Cette silice peut présenter un rapport $V_{(d5 - d50)}/V_{(d5 - d100)}$ d'au moins 0,78, notamment d'au moins 0,80, voire d'au moins 0,84.

Dans les silices selon l'invention (c'est-à-dire conformes à l'une des quatre variantes de l'invention), le volume poreux apporté par les pores les plus gros représente habituellement la plus grande partie de la structure.

Elles peuvent présenter à la fois une largeur Ld de distribution de taille d'objets d'au moins 1,04 et une largeur L'd de distribution de taille d'objets (inférieure à 500 nm) d'au moins 0,95.

La largeur Ld de distribution de taille d'objets des silices selon l'invention peut dans certains cas être d'au moins 1,10, en particulier d'au moins 1,20 ; elle peut être d'au moins 1,30, par exemple d'au moins 1,50, voire d'au moins 1,60.

De même, la largeur L'd de distribution de taille d'objets (inférieure à 500 nm) des silices selon l'invention peut être par exemple d'au moins 1,0, en particulier d'au moins 1,10, notamment d'au moins 1,20.

De préférence, les silices selon l'invention prossèdent une chimie de surface particulière, telle qu'elles présentent un rapport (nombre de Sears x 1000) / (surface spécifique BET ($S_{BET}$)) inférieur à 60, de préférence inférieur à 55, par exemple inférieur à 50.

Les silices selon l'invention présentent généralement une taille d'objets élevée et donc atypique, qui peut être telle que le mode de leur distribution granulométrique mesuré par granulométrie XDC après désagglomération aux ultra-sons (dans l'eau) réponde à la condition : Mode XDC (nm) $\geq$ (5320 $S_{CTAB}$ (m$^2$/g)) + 8, voire à la condition : Mode XDC (nm) $\geq$ (5320 / $S_{CTAB}$ (m$^2$/g)) + 10.

Les silices selon l'invention peuvent posséder par exemple un volume poreux ($V_{80}$) constitué par les pores de diamètres compris entre 3,7 et 80 nm d'au moins 1,35 cm$^3$/g, en particulier d'au moins 1,40 cm$^3$/g, voire d'au moins 1,50 cm$^3$/g.

Les silices selon l'invention présentent de préférence une aptitude satisfaisante à la dispersion (dispersibilité) dans les polymères.

Leur diamètre médian ($\varnothing_{50S}$), après désagglomération aux ultra-sons, est en général inférieur à 8,5 $\mu$m ; il peut être inférieur à 6,0 $\mu$m, par exemple inférieur à 5,5 $\mu$m.

De même, leur diamètre médian ($\varnothing_{50M}$), après désagglomération aux ultra-sons, est en général inférieur à 8,5 $\mu$m ; il peut être inférieur à 6,0 $\mu$m, par exemple inférieur à 5,5 $\mu$m.

Elles peuvent posséder également une vitesse de désagglomération $\alpha$, mesurée au test de désagglomération aux ultra-sons en mode pulsé décrit précédemment, à 100 % de puissance d'une sonde à ultra-sons de 600 watts, d'au moins 0,0035 $\mu$m$^{-1}$.mn$^{-1}$, en particulier d'au moins 0,0037 $\mu$m$^{-1}$.mn$^{-1}$.

Les silices selon l'invention peuvent présenter un facteur de désagglomération aux ultra-sons ($F_{DS}$) supérieur à 3 ml., en particulier supérieur à 3,5 ml, notamment supérieur à 4,5 ml.

Leur facteur de désagglomération aux ultra-sons ($F_{DM}$) peut être supérieur à 6, en particulier supérieur à 7, notamment supérieur à 11.

Les silices selon la présente invention peuvent présenter une taille moyenne (en masse) de particules, mesurée par granulométrise XDC après désagglomération aux ultra-sons, $d_W$, comprise entre 20 et 300 nm, notamment entre 30 et 300 nm, par exemple entre 40 et 160 nm.

Généralement, les silices selon l'invention présentent également au moins l'une, voire l'ensemble, des trois caractéristiques suivantes :

- une distribution de taille de particules telle que $d_w \geq$ (16500 / $S_{CTAB}$) - 30
- une porosité telle que L/IF $\geq$ - 0,0025 $S_{CTAB}$ + 0,85
- un nombre de silanols par unité de surface $N_{SiOH/nm2}$ tel que

$$N_{SiOH/nm2} \leq - 0,027 \, S_{CTAB} + 10,5.$$

Selon un mode de réalisation, les silices selon l'invention présentent en général :

- une surface spécifique CTAB comprise entre 60 et 330 m$^2$/g, en particulier entre 80 et 290 m$^2$/g,
- une surface spécifique BET comprise entre 70 et 350 m$^2$/g, en particulier entre 90 et 320 m$^2$/g.

Leur surface spécifique CTAB peut être comprise entre 90 et 230 m$^2$/g, notamment entre 95 et 200 m$^2$/g, par

exemple entre 120 et 190 m$^2$/g.

De même, leur surface spécifique BET peut être comprise entre 110 et 270 m$^2$/g, notamment entre 115 et 250 m$^2$/g, par exemple entre 135 et 235 m$^2$/g.

Selon un autre mode de réalisation, les silices selon l'invention présentent en général :

- une surface spécifique CTAB comprise entre 40 et 380 m$^2$/g, en particulier entre 45 et 280 m$^2$/g,
- une surface spécifique BET comprise entre 45 et 400 m$^2$/g, en particulier entre 50 et 300 m$^2$/g.

Leur surface spécifique CTAB peut être comprise entre 115 et 260 m$^2$/g, notamment entre 145 et 260 m$^2$/g.

De même, leur surface spécifique BET peut être comprise entre 120 et 280 m$^2$/g, notamment entre 150 et 280 m$^2$/g.

Les silices selon la présente invention peuvent présenter une certaine microporosité ; ainsi, les silices selon l'invention sont habituellement telles que ($S_{BET}$ - $S_{CTAB}$) $\geq$ 5 m$^2$/g, de préférence $\geq$ 15 m$^2$/g, par exemple $\geq$ 25 m$^2$/g.

Cette microporosité n'est pas en général trop importante ; les silices selon l'invention sont généralement telles que ($S_{BET}$ - $S_{CTAB}$) < 50 m$^2$/g, de préférence < 40 m$^2$/g.

Le pH des silices selon l'invention est habituellement compris entre 6,3 et 7,8, notamment entre 6,6 et 7,5.

Elles possèdent une prise d'huile DOP variant, le plus souvent, entre 220 et 330 ml/100g, par exemple entre 240 et 300 ml/100g.

Elles peuvent se présenter sous forme de billes sensiblement sphériques de taille moyenne d'au moins 80 $\mu$m.

Cette taille moyenne des billes peut être d'au moins 100 $\mu$m, par exemple d'au moins 150 $\mu$m ; elle est généralement d'au plus 300 $\mu$m et se situe de préférence entre 100 et 270 $\mu$m. Cette taille moyenne est déterminée selon la norme NF X 11507 (décembre 1970) par tamisage à sec et détermination du diamètre correspondant à un refus cumulé de 50 %.

Les silices selon l'invention peuvent également se présenter sous forme de poudre de taille moyenne d'au moins 15 $\mu$m ; celle-ci est par exemple comprise entre 15 et 60 $\mu$m (notamment entre 20 et 45 $\mu$m) ou entre 30 et 150 $\mu$m (notamment entre 45 et 120 $\mu$m).

Elles peuvent aussi se présenter sous forme de granulés de taille d'au moins 1 mm, en particulier comprise entre 1 et 10 mm, selon l'axe de leur plus grande dimension (longueur).

Les silices selon l'invention sont de préférence préparées selon le procédé de préparation conforme à l'invention et décrit précédemment.

Les silices selon l'invention ou préparées par le procédé selon l'invention trouvent une application particulièrement intéressante dans le renforcement des polymères, naturels ou synthétiques.

Les compositions de polymère(s) dans lesquelles elles sont utilisées, notamment à titre de charge renforçante, sont en général à base d'un ou plusieurs polymères ou copolymères, en particulier d'un ou plusieurs élastomères, notamment les élastomères thermoplastiques, présentant, de préférence, au moins une température de transition vitreuse comprise entre -150 et +300 °C, par exemple entre -150 et +20 °C.

A titre de polymères possibles, on peut citer les polymères diéniques, en particulier les élastomères diéniques.

Par exemple, on peut utiliser les polymères ou copolymères dérivant de monomères aliphatiques ou aromatiques, comprenant au moins une insaturation (tels que, notamment, l'éthylène, le propylène, le butadiène, l'isoprène, le styrène), le polyacrylate de butyle, ou leurs mélanges ; on peut également citer les élastomères silicones, les élastomères fonctionnalisés (par exemple par des fonctions susceptibles de réagir avec la surface de la silice) et les polymères halogénés. On peut mentionner les polyamides.

Le polymère (copolymère) peut être un polymère (copolymère) en masse, un latex de polymère (copolymère) ou bien une solution de polymère (copolymère) dans l'eau ou dans tout autre liquide dispersant approprié.

A titre d'élastomères diéniques, on peut mentionner par exemple les polybutadiènes (BR), les polyisoprènes (IR), les copolymères de butadiène, les copolymères d'isoprène, ou leurs mélanges, et en particulier les copolymères de styrène-butadiène (SBR, notamment ESBR (émulsion) ou SSBR (solution)), les copolymères d'isoprène-butadiène (BIR), les copolymères d'isoprène-styrène (SIR), les copolymères d'isoprène-butadiène-styrène (SBIR), les terpolymères éthylène-propylène-diène (EPDM).

On peut également citer le caoutchouc naturel (NR).

Les compositions de polymère(s) peuvent être vulcanisées au soufre (on obtient alors des vulcanisats) ou réticulées notamment aux péroxydes.

En général, les compositions de polymère(s) comprennent en outre au moins un agent de couplage (silice/polymère) et/ou au moins un agent de recouvrement ; elles peuvent également comprendre, entre autres, un agent antioxydant.

On peut notamment utiliser comme agents de couplage, à titre d'exemples non limitatifs, des silanes polysulfurés, dits "symétriques" ou "asymétriques" ; on peut citer plus particulièrement les polysulfures (notamment disulfures, trisulfures ou tétrasulfures) de bis-(alkoxyl($C_1$-$C_4$)-alkyl($C_1$-$C_4$)silyl-alkyl($C_1$-$C_4$)), comme par exemple les polysulfures de bis(3-(triméthoxysilyl)propyl) ou les polysulfures de bis(3-(triéthoxysilyl)propyl). On peut également citer le tétrasulfure de monoéthoxydiméthylsilylpropyle.

L'agent de couplage peut être préalablement greffé sur le polymère.

Il peut être également employé à l'état libre (c'est-à-dire non préalablement greffé) ou greffé à la surface de la silice. Il en est de même de l'éventuel agent de recouvrement.

L'utilisation d'une silice selon l'invention ou préparée par le procédé selon l'invention peut permettre de réduire sensiblement, par exemple de l'ordre de 20 %, la quantité d'agent de couplage à employer dans des compositions de polymère(s) renforcées par de la silice, tout en maintenant un compromis de propriétés sensiblement identique.

A l'agent de couplage peut éventuellement être associé un "activateur de couplage" approprié, c'est-à-dire un composé qui, mélangé avec cet agent de couplage, augmente l'efficacité de ce dernier.

La proportion en poids de silice dans la composition de polymère(s) peut varier dans une gamme assez large. Elle représente habituellement 20 à 80 %, par exemple 30 à 70 %, de la quantité du (des) polymère (s).

La silice selon l'invention peut avantageusement constituer la totalité de la charge inorganique renforçante, et même la totalité de la charge renforçante, de la composition de polymère(s).

Cependant, à cette silice selon l'invention peut être éventuellement associée au moins une autre charge renforçante, comme en particulier une silice hautement dispersible commerciale telle que par exemple la Z1165MP, la Z1115MP, une silice précipitée traitée (par exemple "dopée" à l'aide d'un cation comme l'aluminium), une autre charge inorganique renforçante telle que par exemple l'alumine, voire même une charge organique renforçante, notamment du noir de carbone (éventuellement recouvert d'une couche inorganique, par exemple de silice). La silice selon l'invention constitue alors de préférence au moins 50 %, voire au moins 80 %, en poids de la totalité de la charge renforçante.

On peut citer, à titre d'exemples, non limitatifs, d'articles finis à base des compositions de polymère(s) décrites précédemment (notamment à base des vulcanisats mentionnés ci-dessus) les semelles de chaussures (de préférence en présence d'un agent de couplage (silice/polymère)), les revêtements de sols, les barrières aux gaz, les matériaux ignifugeants et également les pièces techniques telles que les galets de téléphériques, les joints d'appareils électroménagers, les joints de conduites de liquides ou de gaz, les joints de système de freinage, les gaines, les cables et les courroies de transmissions.

Pour les semelles de chaussures, on peut mettre en oeuvre, de manière avantageuse en présence d'un agent de couplage (silice/polymère), des compositions de polymère(s) à base par exemple de caoutchouc naturel (NR), de polyisoprène (IR), de polybutadiène (BR), de copolymère styrène-butadiène (SBR), de copolymère butadiène-acrylonitrile (NBR).

Pour les pièces techniques, on peut mettre en oeuvre, par exemple en présence d'un agent de couplage (silice/polymère), des compositions de polymère(s) à base par exemple de caoutchouc naturel (NR), de polyisoprène (IR), de polybutadiène (BR), de copolymère styrène-butadiène (SBR), de polychloroprène, de copolymère butadiène-acrylonitrile (NBR), de caoutchouc nitrile hydrogéné ou carboxylé, de copolymère isobutylène-isoprène (IIR), de caoutchouc butyle halogéné (notamment bromé ou chloré), de copolymère éthylène-propylène (EPM), de terpolymère éthylène-propylène-diène (EPDM), de polyéthylène chloré, de polyéthylène chlorosulfoné, de caoutchouc épichlorhydrine, de silicones, de caoutchouc fluorocarboné, de polyacrylates.

Les silices selon l'invention ou préparées par le procédé selon l'invention peuvent être également employées comme support de catalyseur, comme absorbant de matières actives (en particulier support de liquides, par exemple utilisés dans l'alimentation, tels que les vitamines (vitamine E), le chlorure de choline), comme agent viscosant, texturant ou anti-mottant, comme élément pour séparateurs de batteries, comme additif pour dentifrice, pour papier.

Les exemples suivants illustrent l'invention sans toutefois en limiter la portée.

<u>EXEMPLE 1</u>

Dans un réacteur en acier inoxydable de 25 litres, on introduit 10 litres d'eau épurée. La solution est portée à 80 °C. L'ensemble de la réaction est réalisé à cette température. Sous agitation (350 tr/min, agitation hélice), de l'acide sulfurique 80 g/l est introduit jusqu'à ce que le pH atteigne une valeur de 4.

On introduit simultanément dans le réacteur pendant 35 minutes une solution de silicate de sodium (de rapport pondéral $SiO_2/Na_2O$ égal à 3,52) ayant une concentration de 230 g/l à un débit de 76 g/min et de l'acide sulfurique, de concentration égale à 80 g/l, à un débit régulé de manière à maintenir le pH du milieu réactionnel à une valeur de 4. A la 30ème minute de l'addition, la vitesse d'agitation est portée à 450 tr/min.

Au terme des 35 minutes d'addition simultanée, l'introduction d'acide est arrêtée tant que le pH n'a pas atteint une valeur égale à 9. Le débit de silicate est alors également arrêté. On procède à un mûrissement de 15 minutes à pH 9. A la fin du mûrissement, la vitesse d'agitation est amenée à 350 tr/min.

Le pH est ensuite amené à pH 8 par introduction d'acide sulfurique. Une nouvelle addition simultanée est réalisée pendant 40 minutes avec un débit de silicate de sodium de 76 g/min (même silicate de sodium que pour la première addition simultanée) et un débit d'acide sulfurique, de concentration égale à 80g/l, régulé de manière à maintenir le pH du milieu réactionnel à une valeur de 8.

A l'issue de cette addition simultanée, le milieu réactionnel est amené à un pH de 4 par de l'acide sulfurique de concentration égale à 80 g/l. Le milieu est mûri pendant 10 minutes à pH 4. 250 ml de floculant FA 10 (polyoxyéthylène de masse molaire égale à $5.10^6$ g) à 1‰ sont introduits à la 3ème minute du mûrissement.

La bouillie est filtrée et lavée sous vide (extrait sec de 16,7 %). Après dilution (extrait sec de 13 %), le gâteau obtenu est délité mécaniquement. La bouillie résultante est atomisée au moyen d'un atomiseur à turbines.

Les caractéristiques de la silice P1 obtenue sont alors les suivantes :

Surface spécifique CTAB : 221 $m^2$/g
Surface spécifique BET : 240 $m^2$/g
$V_{(d5 - d50)}/V_{(d5 - d100)}$ : 0,74
Largeur Ld (XDC) : 1,62
Largeur de distribution poreuse Idp : 1,42
Largeur L'd (XDC) : 1,27
Nombre de Sears x 1000 / surface spécifique BET : 42,9
Mode XDC : 39 nm
Volume poreux $V_{80}$ : 1,69 $cm^3$/g
$\varnothing_{50S}$ (après désagglomération aux ultra-sons) : 4,8 $\mu$m
$F_{DS}$ : 4,6 ml
$\alpha$ : 0,00626 $\mu m^{-1}.mn^{-1}$
$d_W$ : 79 nm
L/IF : 0,62
$N_{SiOH/nm2}$ : 3,90

## EXEMPLE 2

Dans un réacteur en acier inoxydable de 25 litres, on introduit 9,575 kg d'eau épurée et 522 g de silicate de sodium (de rapport pondéral $SiO_2/Na_2O$ égal à 3,55) ayant une concentration de 235 g/l. La solution est portée à 80 °C. L'ensemble de la réaction est réalisé à cette température. Sous agitation (300 tr/min, agitation hélice), de l'acide sulfurique, de concentration égale à 80 g/l, est introduit jusqu'à ce que le pH atteigne une valeur de 4 (615 g d'acide introduits).

On introduit simultanément dans le réacteur pendant 40 minutes une solution de silicate de sodium (de rapport pondéral $SiO_2/Na_2O$ égal à 3,55) ayant une concentration de 235 g/l à un débit de 50 g/min et de l'acide sulfurique, de concentration égale à 80 g/l, à un débit régulé de manière à maintenir le pH du milieu réactionnel à une valeur de 4.

Au terme des 40 minutes d'addition simultanée, l'introduction d'acide est arrêtée tant que le pH n'a pas atteint une valeur égale à 9. Le débit de silicate est alors également arrêté. On procède à un mûrissement de 15 minutes à pH 9 à 80 °C.

Le pH est ensuite amené à pH 8 en 2 minutes par introduction d'acide sulfurique. Une nouvelle addition simultanée est réalisée pendant 60 minutes avec un débit de silicate de sodium de 76 g/min (même silicate de sodium que pour la première addition simultanée) et un débit d'acide sulfurique, de concentration égale à 80 g/l, régulé de manière à maintenir le pH du milieu réactionnel à une valeur de 8.

A l'issue de cette addition simultanée, le milieu réactionnel est amené à un pH de 4 en 5 minutes par de l'acide sulfurique de concentration égale à 80 g/l. Le milieu est mûri pendant 10 minutes à pH 4.

La bouillie est filtrée et lavée sous vide (extrait sec du gâteau 5,5 %). Après dilution (extrait sec de 12 %), le gâteau obtenu est délité mécaniquement. La bouillie résultante est atomisée au moyen d'un atomiseur à turbines.

Les caractéristiques de la silice P2 obtenue sont alors les suivantes :

Surface spécifique CTAB : 182 $m^2$/g
Surface spécifique BET : 197 $m^2$/g
$V_{(d5 - d50)}/V_{(d5 - d100)}$ : 0,76
Largeur Ld (XDC) : 1,12
Largeur de distribution poreuse Idp : 1,26
Largeur L'd (XDC) : 0,90
Mode XDC : 57 nm
Volume poreux $V_{80}$ : 1,40 $cm^3$/g
$\varnothing_{50S}$ (après désagglomération aux ultra-sons) : 4,1 $\mu$m
$F_{DS}$ : 4,0 ml

EXEMPLE 3

Dans un réacteur en acier inoxydable de 25 litres, on introduit 10 litres de silicate de sodium (de rapport pondéral $SiO_2/Na_2O$ égal à 3,55) ayant une concentration de 10 g/l. La solution est portée à 80 °C. L'ensemble de la réaction est réalisé à cette température. Sous agitation (300 tr/min, agitation hélice), de l'acide sulfurique, de concentration égale à 80 g/l, est introduit jusqu'à ce que le pH atteigne une valeur de 4 (615 g d'acide introduits).

On introduit simultanément dans le réacteur pendant 40 minutes une solution de silicate de sodium (de rapport pondéral $SiO_2/Na_2O$ égal à 3,55) ayant une concentration de 230 g/l à un débit de 50 g/min et de l'acide sulfurique, de concentration égale à 80 g/l, à un débit régulé de manière à maintenir le pH du milieu réactionnel à une valeur de 4.

Au terme des 40 minutes d'addition simultanée, l'introduction d'acide est arrêtée jusqu'à atteindre un pH de 8.

Une nouvelle addition simultanée est réalisée pendant 60 minutes avec un débit de silicate de sodium de 50 g/min (même silicate de sodium que pour la première addition simultanée) et un débit d'acide sulfurique, de concentration égale à 80 g/l, régulé de manière à maintenir le pH du milieu réactionnel à une valeur de 8.

A l'issue de cette addition simultanée, le milieu réactionnel est amené à un pH de 4 en 4 minutes par de l'acide sulfurique de concentration égale à 80 g/l. Le milieu est mûri pendant 10 minutes à pH 4.

La bouillie est filtrée et lavée sous vide (extrait sec du gâteau 13,7 %). Après dilution (extrait sec de 11,2 %), le gâteau obtenu est délité mécaniquement. La bouillie résultante est atomisée au moyen d'un atomiseur à turbines.

Les caractéristiques de la silice P3 sont alors les suivantes :

Surface spécifique CTAB : 228 $m^2$/g
Surface spécifique BET : 245 $m^2$/g
$V_{(d5-d50)}/V_{(d5-d100)}$ : 0,76
Largeur Ld (XDC) : 1,48
Largeur de distribution poreuse ldp : 1,98
Largeur L'd (XDC) : 1,16
Mode XDC : 42 nm
Volume poreux $V_{80}$ : 1,48 $cm^3$/g
$\varnothing_{50S}$ (après désagglomération aux ultra-sons) : 4,4 $\mu$m
$F_{DS}$ : 4,3 ml

EXEMPLE 4

Dans un réacteur en acier inoxydable de 25 litres, on introduit 12 litres d'une solution de silicate de sodium (de rapport pondéral $SiO_2/Na_2O$ égal à 3,5) ayant une concentration de 10 g/l. La solution est portée à 80 °C. L'ensemble de la réaction est réalisé à cette température. Sous agitation (300 tr/min, agitation hélice), de l'acide sulfurique, de concentration égale à 80 g/l, est introduit jusqu'à ce que le pH atteigne une valeur de 8,9.

On introduit simultanément dans le réacteur pendant 15 minutes une solution de silicate de sodium (de rapport pondéral $SiO_2/Na_2O$ égal à 3,5) ayant une concentration de 230 g/l à un débit de 76 g/min et de l'acide sulfurique, de concentration égale à 80 g/l, à un débit régulé de manière à maintenir le pH du milieu réactionnel à une valeur de 8,9. On obtient ainsi un sol de particules peu agrégées. Le sol est soutiré et refroidi rapidement à l'aide d'un serpentin en cuivre dans lequel de l'eau froide circule. Le réacteur est rapidement nettoyé.

Dans le réacteur de 25 litres, 4 litres d'eau épurée sont introduits. De l'acide sulfurique, de concentration égale à 80 g/l, est introduit jusqu'à ce que le pH atteigne une valeur de 4. Une addition simultanée du sol froid à un débit de 195 g/min et d'acide sulfurique, de concentration égale à 80 g/l, à un débit permettant de réguler le pH à 4 est réalisée pendant 40 minutes. Un mûrissement de 10 minutes est réalisé.

Au terme des 40 minutes d'addition simultanée sol/acide sulfurique, une addition simultanée est réalisée pendant 20 minutes avec un débit de silicate de sodium de 76 g/min (même silicate de sodium que pour la première addition simultanée) et un débit d'acide sulfurique 80 g/l régulé de manière à maintenir le pH du milieu réactionnel à une valeur de 4. Au terme des 20 minutes, le débit d'acide est stoppé jusqu'à obtenir un pH de 8.

Une nouvelle addition simultanée est réalisée pendant 60 minutes avec un débit de silicate de sodium de 76 g/min (même silicate de sodium que pour la première addition simultanée) et un débit d'acide sulfurique, de concentration égale à 80 g/l, régulé de manière à maintenir le pH du milieu réactionnel à une valeur de 8. La vitesse d'agitation est augmentée lorsque le milieu devient très visqueux.

A l'issue de cette addition simultanée, le milieu réactionnel est amené à un pH de 4 en 5 minutes par de l'acide sulfurique de concentration égale à 80 g/l. Le milieu est mûri pendant 10 minutes à pH 4.

La bouillie est filtrée et lavée sous vide (extrait sec du gâteau 15 %). Après dilution, le gâteau obtenu est délité mécaniquement. La bouillie résultante est atomisée au moyen d'un atomiseur à turbines.

Les caractéristiques de la silice P4 sont alors les suivantes :

Surface spécifique CTAB : 230 m$^2$/g

Surface spécifique BET : 236 m$^2$/g

$V_{(d5 - d50)}/V_{(d5 - d100)}$ : 0,73

Largeur Ld (XDC) : 1,38

Largeur de distribution poreuse Idp : 0,67

Largeur L'd (XDC) : 1,14

Mode XDC : 34 nm

Volume poreux $V_{80}$ : 1,42 cm$^3$/g

$\varnothing_{50S}$ (après désagglomération aux ultra-sons) : 3,8 $\mu$m

$F_{DS}$ : 4,6 ml

## EXEMPLE 5

Dans un réacteur en acier inoxydable de 25 litres, on introduit 10 litres d'une solution de silicate de sodium (de rapport pondéral $SiO_2/Na_2O$ égal à 3,48) ayant une concentration de 5 g/l. La solution est portée à 80 °C. Sous agitation (300 tr/min, agitation hélice), de l'acide sulfurique, de concentration égale à 80 g/l, est introduit jusqu'à ce que le pH atteigne une valeur de 4,2.

On introduit simultanément dans le réacteur pendant 30 minutes une solution de silicate de sodium (de rapport pondéral $SiO_2/Na_2O$ égal à 3,48) ayant une concentration de 230 g/l à un débit de 75 g/min et de l'acide sulfurique, de concentration égale à 80 g/l, à un débit régulé de manière à maintenir le pH du milieu réactionnel à une valeur de 4,2.

Au terme des 30 minutes d'addition simultanée, l'introduction d'acide est arrêtée tant que le pH n'a pas atteint une valeur égale à 9. Le débit de silicate est alors également arrêté. On procède à un mûrissement de 15 minutes à pH 9, tout en augmentant progressivement la température (en 15 minutes) de 80 à 90 °C, valeur à laquelle la suite de la réaction est effectuée.

Le pH est ensuite amené à pH 8 par introduction d'acide sulfurique de concentration égale à 80 g/l. Une nouvelle addition simultanée est réalisée pendant 50 minutes avec un débit de silicate de sodium de 76 g/min (même silicate de sodium que pour la première addition simultanée) et un débit d'acide sulfurique, de concentration égale à 80 g/l, régulé de manière à maintenir le pH du milieu réactionnel à une valeur de 8.

A l'issue de cette addition simultanée, le milieu réactionnel est amené à un pH de 4 par de l'acide sulfurique de concentration égale à 80 g/l. Le milieu est mûri pendant 10 minutes à pH 4.

La bouillie est filtrée et lavée sous vide (extrait sec du gâteau 19,6 %). Après dilution (extrait sec de 10 %), le gâteau obtenu est délité mécaniquement. La bouillie résultante est atomisée au moyen d'un atomiseur à turbines.

Les caractéristiques de la silice P5 obtenue sont alors les suivantes :

Surface spécifique CTAB : 135 m$^2$/g

Surface spécifique BET : 144 m$^2$/g

$V_{(d5 - d50)}/V_{(d5 - d100)}$ : 0,76

Largeur Ld (XDC) : 1,52

Largeur de distribution poreuse Idp : 2,65

Largeur L'd (XDC) : 0,92

Nombre de Sears x 1000 / surface spécifique BET : 49,3

Mode XDC : 57 nm

Volume poreux $V_{80}$ : 1,12 cm$^3$/g

$\varnothing_{50S}$ (après désagglomération aux ultra-sons) : 5,9 $\mu$m

$d_w$ : 159 nm

L/IF : 1,47

$N_{SiOH/nm2}$ : 5,20

## EXEMPLE 6

On prépare trois compositions de polymère :

- l'une contenant de la silice précipitée haute dispersibilité Z1165MP commercialisée par la société Rhodia, de densité de 2,1 g/cm$^3$, et un agent de couplage (composition de référence R1),
- les deux autres contenant chacune de la silice préparée dans l'exemple 4 et un agent de couplage (compositions C1 et C2).

La silice Z1165MP présente les caractéristiques suivantes :

Surface spécifique CTAB : 160 m$^2$/g
Largeur Ld (XDC) : 0,56
Largeur de distribution poreuse Idp : 0,50
Largeur L'd (XDC) : 0,56
Mode XDC : 41 nm
Volume poreux $V_{80}$ : 1,12 cm$^3$/g
$\varnothing_{50S}$ (après désagglomération aux ultra-sons) < 6 $\mu$m
$\alpha$ : 0,0049 $\mu$m$^{-1}$.mn$^{-1}$
$d_w$ : 59 nm
L/IF : 0,39
$N_{SiOH/nm2}$ : 8,10

Tableau 1

| (compositions en parties, en poids) | | | |
|---|---|---|---|
| | Composition R1 | Composition C1 | Composition C2 |
| SBR [1] | 100 | 100 | 100 |
| Silice Z1165MP | 50 | 0 | 0 |
| Silice Exemple 4 | 0 | 50 | 50 |
| Silane Si69 [2] | 4 | 4 | 6,25 |
| Diphénylquanidine | 1,45 | 1,45 | 1,45 |
| Acide stéarique | 1,1 | 1,1 | 1,1 |
| Oxyde de zinc | 1,82 | 1,82 | 1,82 |
| Anti-oxydant [3] | 1,45 | 1,45 | 1,45 |
| Sulfénamide [4] | 1,3 | 1,3 | 1,3 |
| Soufre | 1,1 | 1,1 | 1,1 |
| (1) Copolymère styrène butadiène synthétisé en solution (type Buna VSL 5525-0) non étendu à l'huile<br>(2) Agent de couplage charge/polymère (commercialisé par la société Degussa)<br>(3) N-(1,3-diméthyl-butyl)-N'-phényl-p-phénylènediamine<br>(4) N-cyclohexyl-2-benzothiazyl sulfénamide (CBS) | | | |

La composition C1 contient une quantité d'agent de couplage identique à celle de la composition de référence R1. La composition C2 contient une quantité d'agent de couplage optimisée par rapport à la surface spécifique de la silice utilisée (exemple 4).

Les compositions sont préparées en travaillant thermo-mécaniquement les élastomères dans un malaxeur interne (type BRABENDER) d'un volume égal à 75 cm$^3$, en deux étapes, avec une vitesse moyenne des palettes de 50 tours/minute, jusqu'à l'obtention d'une température de 120 °C, ces étapes étant suivies d'une étape de finition réalisée sur un mélangeur externe.

La température de vulcanisation est choisie à 170 °C. Les conditions de vulcanisation des compositions sont adaptées aux cinétiques de vulcanisation des mélanges correspondants.

Les propriétés des compositions sont exposées ci-après, les mesures ayant été effectuées (sur les compositions vulcanisées), selon les normes et/ou méthodes suivantes :

Propriétés de vulcanisation (propriétés rhéologiques)
(Propriétés à cru - Rhéométrie à 170 °C, t = 30 minutes)
Norme NF T 43015

On utilise, notamment pour la mesure du couple mini (Cmin) et du couple maxi (Cmax), un rhéomètre Monsanto 100 S.

Ts2 correspond au temps pendant lequel le contrôle du mélange est possible ; le mélange de polymère durcit à partir de Ts2 (début de la vulcanisation).

T90 correspond au temps au bout duquel 90 % de la vulcanisation ont été réalisés.

Propriétés mécaniques (des compositions vulcanisées à 170 °C)

Traction (modules) : norme NF T 46002

Les modules x % correspondent à la contrainte mesurée à x % de déformation en traction.

Tableau 2

|  | Composition R1 | Composition C1 | Composition C2 |
|---|---|---|---|
| Vulcanisation |  |  |  |
| Cmin (In.Ib) | 10 | 21 | 14 |
| Ts2 (min) | 3,1 | 2,1 | 3,1 |
| T90 (min) | 29,4 | 42,0 | 36,4 |
| Cmax (In.Ib) | 91 | 97,5 | 103 |
| Mécanique |  |  |  |
| Module 10 % (MPa) | 0,95 | 1,3 | 1,05 |
| Module 100 % (MPa) | 3,6 | 4,0 | 4,6 |
| Module 200 % (MPa) | 9,5 | 9,8 | 12,2 |

On constate que les compositions C1 et C2 contenant une silice selon l'invention présentent un compromis de propriétés intéressant par rapport à celui de la composition de référence R1.

Malgré des conditions de vulcanisation non optimisées, la composition C1 conduit à un renforcement en termes de modules plus marqué que la composition de référence R1.

L'ajustement du taux d'agent de couplage effectué dans le cas de la composition C2 conduit à une cinétique de vulcanisation comparable à celle de la composition de référence R1 ; de plus, la composition C2 présentent des modules (en particulier modules 100 % et 200 %) très supérieurs à ceux obtenus avec la composition de référence R1.

EXEMPLE 7

Dans un réacteur en acier inoxydable de 25 litres, on introduit 10 litres d'une solution de silicate de sodium (de rapport pondéral $SiO_2/Na_2O$ égal à 3,53) ayant une concentration de 5 g/l. La solution est portée à 80 °C. Sous agitation (300 tr/min, agitation hélice), de l'acide sulfurique, de concentration égale à 80 g/l, est introduit jusqu'à ce que le pH atteigne une valeur de 4,2.

On introduit simultanément dans le réacteur pendant 35 minutes une solution de silicate de sodium (de rapport pondéral $SiO_2/Na_2O$ égal à 3,53) ayant une concentration de 230 g/l à un débit de 50 g/min et de l'acide sulfurique, de concentration égale à 80 g/l, à un débit régulé de manière à maintenir le pH du milieu réactionnel à une valeur de 4,2.

Au terme des 35 minutes d'addition simultanée, l'introduction d'acide est arrêtée tant que le pH n'a pas atteint une valeur égale à 9. Le débit de silicate est alors également arrêté. On procède à un mûrissement de 15 minutes à pH 9, tout en augmentant progressivement la température (en 15 minutes) de 80 à 90 °C, valeur à laquelle la suite de la réaction est effectuée.

Le pH est ensuite amené à pH 8 par introduction d'acide sulfurique de concentration égale à 80 g/l. Une nouvelle addition simultanée est réalisée pendant 50 minutes avec un débit de silicate de sodium de 50 g/min (même silicate de sodium que pour la première addition simultanée) et un débit d'acide sulfurique, de concentration égale à 80 g/l, régulé de manière à maintenir le pH du milieu réactionnel à une valeur de 8.

A l'issue de cette addition simultanée, le milieu réactionnel est amené à un pH de 4 par de l'acide sulfurique de concentration égale à 80 g/l. Le milieu est mûri pendant 10 minutes à pH 4.

La bouillie est filtrée et lavée sous vide (extrait sec du gâteau 16,8 %). Après dilution (extrait sec de 10 %), le gâteau obtenu est délité mécaniquement. La bouillie résultante est atomisée au moyen d'un atomiseur à turbines.

Les caractéristiques de la silice P6 obtenue sont alors les suivantes :

Surface spécifique CTAB : 170 $m^2$/g
Surface spécifique BET : 174 $m^2$/g

$V_{(d5 - d50)}/V_{(d5 - d100)}$ : 0,78
Largeur Ld (XDC) : 3,1
Largeur de distribution poreuse Idp : 1,42
Largeur L'd (XDC) : 2,27
Nombre de Sears x 1000 / surface spécifique BET : 50,6
Mode XDC : 41 nm
Volume poreux $V_{80}$ : 1,38 cm$^3$/g
$\varnothing_{50S}$ (après désagglomération aux ultra-sons) : 4,3 $\mu$m
$F_{DS}$ : 3,7 ml
$\alpha$ : 0,00883 $\mu$m$^{-1}$.mn$^{-1}$
$d_w$ : 98nm
L/IF : 0,78
$N_{SiOH/nm2}$ : 4,40

EXEMPLE 8

Dans un réacteur de 2000 litres, on introduit 700 litres d'eau industrielle. Cette solution est portée à 80 °C par chauffage par injection directe de vapeur. Sous agitation (95 tr/min), de l'acide sulfurique, de concentration égale à 80 g/l, est introduit jusqu'à ce que le pH atteigne une valeur de 4.

On introduit simultanément dans le réacteur pendant 35 minutes une solution de silicate de sodium (de rapport pondéral SiO$_2$/Na$_2$0 égal à 3,52) ayant une concentration de 230 g/l à un débit de 190 l/heure et de l'acide sulfurique, de concentration égale à 80 g/l, à un débit régulé de manière à maintenir le pH du milieu réactionnel à une valeur de 4.

Au terme des 35 minutes d'addition simultanée, l'introduction d'acide est arrêtée tant que le pH n'a pas atteint une valeur égale à 8. Une nouvelle addition simultanée est ensuite réalisée pendant 40 minutes avec un débit de silicate de sodium de 190 l/heure (même silicate de sodium que pour la première addition simultanée) et un débit d'acide sulfurique, de concentration égale à 80 g/l, régulé de manière à maintenir le pH du milieu réactionnel à une valeur de 8.

A l'issue de cette addition simultanée, le milieu réactionnel est amené à un pH de 5,2 par de l'acide sulfurique de concentration égale à 80 g/l. Le milieu est mûri pendant 5 minutes à pH 5,2.

La bouillie est filtrée et lavée sous filtre presse (extrait sec du gâteau 22 %). Le gâteau obtenu est délité en ajoutant une quantité d'aluminate de sodium correspondant à un rapport pondéral Al/SiO$_2$ de 0,3 %. La bouillie résultante est atomisée au moyen d'un atomiseur à buses.

Les caractéristiques de la silice obtenue P7 sous forme de billes sensiblement sphériques sont alors les suivantes :

Surface spécifique CTAB : 200 m$^2$/g
Surface spécifique BET : 222 m$^2$/g
$V_{(d5 - d50)}/V_{(d5 - d100)}$ : 0,71
Largeur Ld (XDC) : 1,0
Largeur de distribution poreuse Idp : 1,51
Largeur L'd (XDC) : 0,93
Nombre de Sears x 1000 / surface spécifique BET : 31,5
Mode XDC : 34 nm
Volume poreux $V_{80}$ : 1,44 cm$^3$/g
Taille moyenne des particules : > 150 $\mu$m
$\varnothing_{50S}$ (après désagglomération aux ultra-sons) : 4,8 $\mu$m
$F_{DS}$ : 5,4 ml
$\varnothing_{50M}$ (après désagglomération aux ultra-sons) : 5,0 $\mu$m
$F_{DM}$ : 11,5
$\alpha$ : 0,00566 $\mu$m$^{-1}$.mn$^{-1}$
$d_w$ : 68 nm
L/IF : 0,70
$N_{SiOH/nm2}$ : 4,50

EXEMPLE 9

Dans un réacteur de 2000 litres, on introduit 700 litres d'eau industrielle. Cette solution est portée à 78 °C par chauffage par injection directe de vapeur. Sous agitation (95 tr/min), de l'acide sulfurique, de concentration égale à 80 g/l, est introduit jusqu'à ce que le pH atteigne une valeur de 4.

On introduit simultanément dans le réacteur pendant 35 minutes une solution de silicate de sodium (de rapport pondéral SiO$_2$/Na$_2$0 égal à 3,52) ayant une concentration de 230 g/l à un débit de 190 l/heure et de l'acide sulfurique, de concentration égale à 80 g/l, à un débit régulé de manière à maintenir le pH du milieu réactionnel à une valeur de 4.

Au terme des 35 minutes d'addition simultanée, l'introduction d'acide est arrêtée tant que le pH n'a pas atteint une valeur égale à 8. Une nouvelle addition simultanée est ensuite réalisée pendant 40 minutes avec un débit de silicate de sodium de 190 l/heure (même silicate de sodium que pour la première addition simultanée) et un débit d'acide sulfurique, de concentration égale à 80 g/l, régulé de manière à maintenir le pH du milieu réactionnel à une valeur de 8.

A l'issue de cette addition simultanée, le milieu réactionnel est amené à un pH de 5,2 par de l'acide sulfurique de concentration égale à 80 g/l. Le milieu est mûri pendant 5 minutes à pH 5,2.

La bouillie est filtrée et lavée sous filtre sous-vide (extrait sec du gâteau 18 %). Le gâteau obtenu est délité mécaniquement à l'aide d'eau industrielle (10 % d'eau ajoutée par rapport au gâteau) en ajoutant une quantité d'aluminate de sodium correspondant à un rapport pondéral Al/SiO$_2$ de 0,3 %. La bouillie résultante est atomisée au moyen d'un atomiseur à turbines.

Les caractéristiques de la silice obtenue P8 sont alors les suivantes :

Surface spécifique CTAB : 194 m$^2$/g
Surface spécifique BET : 212 m$^2$/g
V$_{(d5 - d50)}$/V$_{(d5 - d100)}$ : 0,75
Largeur Ld (XDC) : 1,11
Largeur de distribution poreuse Idp : 0,83
Largeur L'd (XDC) : 4,29
Nombre de Sears x 1000 / surface spécifique BET : 34,9
Mode XDC : 47 nm
Volume poreux V$_{80}$ : 1,37 cm$^3$/g
$\varnothing_{50S}$ (après désagglomération aux ultra-sons) : 5,9 $\mu$m
$\alpha$ : 0,00396 $\mu$m$^{-1}$.mn$^{-1}$

EXEMPLE 10

On prépare deux compositions de polymère :
- l'une contenant de la silice précipitée haute dispersibilité Z1165MP commercialisée par la société Rhodia (caractéristiques mentionnées dans l'exemple 6) et un agent de couplage (composition de référence R2),
- l'autre contenant de la silice préparée dans l'exemple 8 et un agent de couplage (composition C3).

Tableau 3

| (compositions en parties, en poids) | | |
|---|---|---|
| | Composition R2 | Composition C3 |
| BR [1] | 70 | 70 |
| SBR [2] | 15 | 15 |
| NBR [3] | 15 | 15 |
| Silice Z1165MP | 50 | 0 |
| Silice Exemple 8 | 0 | 50 |
| Silquest A1891 [4] | 1 | 1 |
| Huile paraffinique [5] | 10 | 10 |
| Acide stéarique | 1,5 | 1,5 |
| Oxyde de zinc | 3 | 3 |
| Polyéthylène glycol [6] | 3 | 3 |
| TBBS [7] | 1 | 1 |
| TBzTD [8] | 0,6 | 0,6 |

(suite)

| (compositions en parties, en poids) | | |
|---|---|---|
| | Composition R2 | Composition C3 |
| Soufre | 1,5 | 1,5 |

| |
|---|
| (1) Polybutadiène (type Kosyn KBR01) |
| (2) Copolymère styrène butadiène synthétisé en solution (type Buna VSL 5025) non étendu à l'huile |
| (3) Copolymère butadiène acrylonitrile (type Krynac 34-50) |
| (4) Agent de couplage charge/polymère, γ-mercaptopropyltriethoxysilane (commercialisé par la société Crompton) |
| (5) Plastol 352 (commercialisée par la société Exxon) |
| (6) Type PEG 4000 (commercialisé par la société Hüls) |
| (7) N-ter-butylbenzothiazyl sulfénamide |
| (8) Disulfure de tétrabenzylthiurame |

Les compositions sont préparées en travaillant thermo-mécaniquement les élastomères dans un malaxeur interne (type BANBURY) d'un volume égal à 1200 cm$^3$. La température initiale et la vitesse des rotors sont fixées de manière à atteindre des températures de tombée de mélange voisines de 120 °C. Cette étape est suivie d'une étape de finition réalisée sur un mélangeur externe à des températures inférieures à 110 °C. Cette phase permet l'introduction du système de vulcanisation.

La température de vulcanisation est choisie à 160 °C. Les conditions de vulcanisation des compositions sont adaptées aux cinétiques de vulcanisation des mélanges correspondants.

Les propriétés des compositions sont exposées ci-après, les mesures ayant été effectuées selon les normes et/ou méthodes suivantes :

Propriétés de vulcanisation (propriétés rhéologiques)
(Propriétés à cru - Rhéométrie à 160 °C, t = 30 minutes)
Norme NF T 43015

On utilise, notamment pour la mesure du couple mini (Cmin) et du couple maxi (Cmax), un rhéomètre Monsanto 100 S.

Ts2 correspond au temps pendant lequel le contrôle du mélange est possible ; le mélange de polymère durcit à partir de Ts2 (début de la vulcanisation).

T90 correspond au temps au bout duquel 90 % de la vulcanisation ont été réalisés.

Propriétés mécaniques (des compositions vulcanisées à 160 °C)

[0226] - Traction (modules, résistance à la rupture, allongement à la rupture) : norme NF T 46002
Les modules x % correspondent à la contrainte mesurée à x % de déformation en traction.
- Résistance au déchirement : norme NF T 46007 (méthode B).
- Dureté Shore A: norme ASTM D2240 ; la valeur considérée est mesurée 15 secondes après l'application de la force.
- Résistance à l'abrasion : norme DIN 53516 ; la valeur mesurée est la perte à l'abrasion : plus elle est faible, meilleure est la résistance à l'abrasion.

Tableau 4

| | Composition R2 | Composition C3 |
|---|---|---|
| Vulcanisation | | |
| Cmin (In.lb) | 22 | 28 |
| Ts2 (min) | 0,8 | 1,4 |
| T90 (min) | 3,3 | 2,8 |
| Cmax (In.lb) | 96 | 95 |
| Mécanique | | |
| Module 10 % (MPa) | 0,8 | 0,8 |

(suite)

|  | Composition R2 | Composition C3 |
|---|---|---|
| Module 100 % (MPa) | 2,8 | 3,1 |
| Module 300 % (MPa) | 9,0 | 8,9 |
| Résistance rupture (MPa) | 11,9 | 12,8 |
| Allongement rupture (%) | 377 | 418 |
| Résistance déchirement (ent n°10)(Kn/m) | 68 | 73 |
| Dureté Shore A (pts) | 68 | 70 |
| Perte abrasion (mm$^3$) | 36 | 29 |

On constate que la composition C3 contenant une silice selon l'invention présente un compromis de propriétés particulièrement intéressant par rapport à celui de la composition de référence R2.

Tout en ayant une cinétique de vulcanisation comparable à celle de la composition de référence R2 et des modules voisins de ceux de la composition de référence R2, la composition C3 possède une résistance à la rupture, un allongement à la rupture, une résistance au déchirement et une dureté Shore supérieurs à ceux de la composition de référence R2. Et surtout, la composition C3 présente une résistance à l'abrasion beaucoup plus élevée que la composition de référence R2 : la perte à l'abrasion est ainsi réduite de près de 20 %.

EXEMPLE 13

On prépare trois compositions de polymère :
- l'une contenant de la silice précipitée haute dispersibilité Z1165MP commercialisée par la société Rhodia (caractéristiques mentionnées dans l'exemple 6) et un agent de couplage (composition de référence R3),
- les deux autres contenant de la silice préparée dans l'exemple 8 et un agent de couplage (composition C4), ou de la silice préparée dans l'exemple 9 et un agent de couplage (composition C5).

Tableau 5

| (compositions en parties, en poids) | | | |
|---|---|---|---|
|  | Composition R3 | Composition C4 | Composition C5 |
| SBR [1] | 103 | 103 | 103 |
| BR [2] | 25 | 25 | 25 |
| Silice Z1165MP | 80 | 0 | 0 |
| Silice Exemple 8 | 0 | 80 | 0 |
| Silice Exemple 9 | 0 | 0 | 80 |
| TESPT [3] | 6,4 | 8,0 | 7,7 |
| Acide stéarique | 2,0 | 2,0 | 2,0 |
| Oxyde de zinc | 2,5 | 2,5 | 2,5 |
| Antioxydant [4] | 1,9 | 1,9 | 1,9 |
| DPG [5] | 1,5 | 1,8 | 1,8 |
| CBS [6] | 2,0 | 2,0 | 2,0 |

(suite)

| (compositions en parties, en poids) | | |
|---|---|---|
| | Composition R3 | Composition C4 | Composition C5 |
| Soufre | 1,1 | 1,1 | 1,1 |

(1) Copolymère styrène butadiène synthétisé en solution (type Buna VSL 5025-1) étendu à l'huile (37,5 % en poids)
(2) Polybutadiène (type Buna CB24 commercialisé par la société Bayer)
(3) Agent de couplage charge/polymère : tétrasulfure de bis(3-(triéthoxysilyl)propyl) (commercialisé par la société Degussa sous la dénomination Si69)
(4) N-1,3-diméthylbutyl-N-phényl-para-phénylènediamine (Santoflex 6-PPD commercialisé par la société Flexsys)
(5) Diphénylguanidine (Vulkacit D commercialisé par la société Bayer)
(6) N-cyclohexyl-2-benzothiazyl-sulfénamide (Santocure commercialisé par la société Flexsys)

Chacune des trois compositions est préparée en trois phases successives. Les deux premières phases réalisées dans un mélangeur interne permettent un travail thermo-mécanique à haute température jusqu'à atteindre une température maximum voisine de 150 °C. Elles sont suivies d'une troisième phase de travail mécanique effectuée sur cylindres à des températures inférieures à 110 °C. Cette dernière phase permet l'introduction du système de vulcanisation.

Le mélangeur employé pour les deux premières phases est un mélangeur interne de type BRABENDER, d'une capacité de 70 cm$^3$. La température initiale et la vitesse des rotors sont fixées à chaque fois de manière à atteindre des températures de tombée de mélange voisines de 150 °C.

La première étape permet d'incorporer les élastomères (à $t_0$), la silice (introduction fractionnée, 2/3 puis 1/3) avec l'agent de couplage (à $t_0$ + 2 mn) puis avec la DPG (à $t_0$ + 4 mn), et enfin l'acide stéarique (à $t_0$ + 6 mn). Après décharge du mélangeur (tombée du mélange, à $t_0$ + 7 mn), puis refroidissement du mélange (température inférieure à 100 °C) et réintroduction (à $t'_0$) dans le mélangeur interne (la température montant ensuite progressivement), une seconde étape dans ce mélangeur permet par un traitement thermo-mécanique d'améliorer la dispersion de la silice et de son agent de couplage dans la matrice élastomérique. Au cours de cette étape, on incorpore (à $t'_0$ + 1 mn) l'oxyde de zinc et l'agent antioxydant.

Après décharge du mélangeur (tombée du mélange, à $t'_0$ + 4 mn), puis refroidissement du mélange (température inférieure à 100 °C), la troisième phase permet l'introduction du système de vulcanisation (soufre et CBS). Elle est réalisée sur un mélangeur à cylindres, préchauffé à 50 °C. La durée de cette phase est comprise entre 5 et 20 minutes.

Après homogénéisation et passages au fin, chaque mélange final est calandré sous la forme de plaques d'épaisseur de 2-3 mm.

La température de vulcanisation est choisie à 160 °C. Les conditions de vulcanisation des compositions sont adaptées aux cinétiques de vulcanisation des mélanges correspondants.

Les propriétés des compositions sont exposées ci-après, les mesures ayant été effectuées selon les normes et/ou méthodes indiquées dans l'exemple 10.

Les propriétés dynamiques (des compositions vulcanisées à 160 °C), comme la tangente delta à 60 °C, sont déterminées sur un viscoélasticimètre Metravib VA3000, selon la norme ASTM D5992, avec une pré-contrainte de 4 %, une fréquence de 10 Hz (onde sinusoïdale).

Tableau 6

| | Composition R3 | Composition C4 | Composition C5 |
|---|---|---|---|
| Vulcanisation | | | |
| Cmin (daN.m) | 25 | 33 | 27 |
| Ts2 (min) | 3,9 | 3,8 | 4,1 |
| T90 (min) | 14,2 | 16,3 | 15,2 |
| Cmax (daN.m) | 71 | 76 | 75 |
| Mécanique | | | |
| Module 10 % (MPa) | 0,6 | 0,7 | 0,6 |
| Module 100 % (MPa) | 2,4 | 2,8 | 2,9 |
| Module 200 % (MPa) | 6,4 | 7,4 | 7,2 |

(suite)

|  | Composition R3 | Composition C4 | Composition C5 |
|---|---|---|---|
| Dureté Shore A (pts) | 62 | 67 | 67 |
| Perte abrasion (mm$^3$) | 72 | 56 | 58 |
| Dynamique |  |  |  |
| Tan delta (60 °C) | 0,121 | 0,113 | 0,100 |

On constate que les compositions C4 et C5 contenant chacune une silice selon l'invention présentent un compromis de propriétés particulièrement intéressant par rapport à celui de la composition de référence R3.

Tout en ayant une cinétique de vulcanisation comparable à celle de la composition de référence R3, les composition C4 et C5 possèdent des modules et une dureté Shore supérieurs à ceux de la composition de référence R3. Et surtout, les compositions C4 et C5 présentent une résistance à l'abrasion bien plus élevée que la composition de référence R3 : la perte à l'abrasion est ainsi réduite d'environ 20 %. Enfin, les compositions C4 et C5 possèdent une tangente delta à 60 °C plus faible que celle de la composition de référence R3, ce qui se révèle aussi particulièrement intéressant pour les propriétés des articles finis à base de ces compositions C4 ou C5.

**Revendications**

1. Silice **caractérisée en ce qu'**elle possède :

 - une surface spécifique CTAB ($S_{CTAB}$) comprise entre 95 et 525 m$^2$/g,
 - une surface spécifique BET ($S_{BET}$) comprise entre 45 et 550 m$^2$/g,
 - une largeur Ld ((d84 - d16)/d50) de distribution de taille d'objets mesurée par granulométrie XDC après désagglomération aux ultra-sons d'au moins 0,91 et
 - une répartition du volume poreux telle que le rapport $V_{(d5 - d50)}/V_{(d5 - d100)}$ est d'au moins 0,66, en particulier d'au moins 0,68.

2. Silice selon la revendication 1, **caractérisée en ce qu'**elle possède une largeur Ld de distribution de taille d'objets d'au moins 0,94.

3. Silice selon l'une des revendications 1 et 2, **caractérisée en ce qu'**elle possède:

 - une largeur Ld ((d84 - d16)/d50) de distribution de taille d'objets mesurée par granulométrie XDC après désagglomération aux ultra-sons d'au moins 1,04 et
 - une répartition du volume poreux telle que le rapport $V_{(d5 - d50)}/V_{(d5 - d100)}$ est d'au moins 0,71.

4. Silice selon l'une des revendications 1 à 3, **caractérisée en ce qu'**elle présente un diamètre médian Sympatec ($\varnothing_{50S}$), après désagglomération aux ultra-sons, inférieur à 8,5 $\mu$m, en particulier inférieur à 6,0 $\mu$m.

5. Silice selon l'une des revendications 1 à 4, **caractérisée en ce qu'**elle présente un diamètre médian Malvern ($\varnothing_{50M}$), après désagglomération aux ultra-sons, inférieur à 8,5 $\mu$m, en particulier inférieur à 6,0 $\mu$m.

6. Silice selon l'une des revendications 1 à 3, **caractérisée en ce que** son rapport $V_{(d5 - d50)}/V_{(d5 - d100)}$ est d'au moins 0,73, en particulier d'au moins 0,74.

7. Silice selon l'une des revendications 1 à 6, **caractérisée en ce qu'**elle présente un rapport (nombre de Sears x 1000) / (surface spécifique BET ($S_{BET}$)) inférieur à 60, de préférence inférieur à 55.

8. Silice selon l'une des revendications 1 à 7, **caractérisée en ce qu'**elle possède un volume poreux ($V_{80}$) constitué par les pores de diamètres compris entre 3,7 et 80 nm d'au moins 1,35 cm$^3$/g.

9. Silice selon l'une des revendications 1 à 8, **caractérisée en ce qu'**elle possède :

 - une surface spécifique CTAB ($S_{CTAB}$) comprise entre 95 et 330 m$^2$/g, en particulier entre 95 et 290 m$^2$/g,

- une surface spécifique BET ($S_{BET}$) comprise entre 70 et 350 m$^2$/g, en particulier entre 90 et 320 m$^2$/g.

**10.** Silice selon l'une des revendications 1 à 9, **caractérisée en ce qu'**elle présente une surface spécifique BET ($S_{BET}$) comprise entre 110 et 270 m$^2$/g, notamment entre 115 et 250 m$^2$/g.

**11.** Silice selon l'une des revendications 1 à 10, **caractérisée en ce que** ($S_{BET}$ - $S_{CTAB}$) $\geq$ 5 m$^2$/g, de préférence $\geq$ 15 m$^2$/g.

**12.** Silice selon l'une des revendications 1 à 11 **caractérisée en ce que** ($S_{BET}$ - $S_{CTAB}$) < 50 m$^2$/g, de préférence < 40 m$^2$/g.

**13.** Utilisation comme charge renforçante pour polymères d'une silice selon l'une des revendications 1 à 12.

**14.** Utilisation comme charge renforçante dans une composition de caoutchouc naturel d'une silice selon l'une des revendications 1 à 12.

**15.** Utilisation d'une silice selon l'une des revendications 1 à 12 comme support de catalyseur, comme absorbant de matières actives, comme agent viscosant, texturant ou anti-mottant, comme élément pour séparateurs de batteries, comme additif pour dentifrice, pour papier.

Figure 1:

Europäisches
Patentamt

European
Patent Office

Office européen
des brevets

**RAPPORT DE RECHERCHE EUROPEENNE**

Numéro de la demande

EP 16 16 3156

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 0 745 558 A2 (DEGUSSA [DE])<br>4 décembre 1996 (1996-12-04)<br>* revendication 2 *<br>* page 4, ligne 44 - ligne 45 *<br>* exemples 1-4 *<br>----- | 1-15 | INV.<br>C01B33/193<br>C08K3/36<br>C08L21/00<br>H01M2/16 |
| A | WO 97/46485 A1 (RHONE POULENC CHIMIE [FR]; AMICHE FREDERIC [FR]; DROMARD ADRIEN [FR])<br>11 décembre 1997 (1997-12-11)<br>* revendications 3,16 *<br>* page 4, ligne 9 - ligne 35 *<br>* page 5, ligne 7 - ligne 6 *<br>----- | 1-15 | |
| A | WO 01/07364 A (DOKTER WILLEM HENDRIK ;AKZO PQ SILICA VOF (NL); TIJBURG IVO IGNATI)<br>1 février 2001 (2001-02-01)<br>* revendications 1-8 *<br>----- | 1-15 | |
| A | WO 98/54090 A (CHEVALLIER YVONNICK ;COCHET PHILIPPE (FR); FOURRE PATRICK (FR); RH)<br>3 décembre 1998 (1998-12-03)<br>* revendications 1-26 *<br>----- | 1-15 | DOMAINES TECHNIQUES RECHERCHES (IPC) |
| X | EP 0 520 862 A (RHONE POULENC CHIMIE)<br>30 décembre 1992 (1992-12-30)<br><br>* revendications 1-4,16-41 *<br>----- | 1-3,<br>6-10,13,<br>14 | C01B<br>C08K<br>A61K<br>H01M |

Le présent rapport a été établi pour toutes les revendications

| Lieu de la recherche | Date d'achèvement de la recherche | Examinateur |
|---|---|---|
| Munich | 16 juin 2016 | Zimpfer, Emmanuel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 16 16 3156

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-06-2016

| Document brevet cité au rapport de recherche | | | Date de publication | Membre(s) de la famille de brevet(s) | | | Date de publication |
|---|---|---|---|---|---|---|---|
| EP 0745558 | A2 | | 04-12-1996 | BR | 9602592 | A | 06-10-1998 |
| | | | | EP | 0745558 | A2 | 04-12-1996 |
| | | | | ES | 2133864 | T3 | 16-09-1999 |
| | | | | ID | 16472 | A | 02-10-1997 |
| | | | | IN | 188702 | B | 26-10-2002 |
| | | | | JP | 3037615 | B2 | 24-04-2000 |
| | | | | JP | H0925115 | A | 28-01-1997 |
| | | | | PL | 314565 | A1 | 09-12-1996 |
| | | | | TR | 9600452 | A2 | 21-12-1996 |
| | | | | TW | 420702 | B | 01-02-2001 |
| | | | | US | 5705137 | A | 06-01-1998 |
| | | | | US | 5859117 | A | 12-01-1999 |
| WO 9746485 | A1 | | 11-12-1997 | AT | 222566 | T | 15-09-2002 |
| | | | | AU | 730648 | B2 | 08-03-2001 |
| | | | | BG | 102961 | A | 30-07-1999 |
| | | | | BR | 9709522 | A | 10-08-1999 |
| | | | | CA | 2257184 | A1 | 11-12-1997 |
| | | | | CN | 1221393 | A | 30-06-1999 |
| | | | | DE | 69714846 | D1 | 26-09-2002 |
| | | | | DE | 69714846 | T2 | 28-05-2003 |
| | | | | EP | 0907612 | A1 | 14-04-1999 |
| | | | | ES | 2182096 | T3 | 01-03-2003 |
| | | | | FR | 2749576 | A1 | 12-12-1997 |
| | | | | JP | 3431168 | B2 | 28-07-2003 |
| | | | | JP | 2000502315 | A | 29-02-2000 |
| | | | | KR | 20000016390 | A | 25-03-2000 |
| | | | | PL | 330144 | A1 | 26-04-1999 |
| | | | | TR | 9802525 | T2 | 21-04-1999 |
| | | | | US | 6187292 | B1 | 13-02-2001 |
| | | | | WO | 9746485 | A1 | 11-12-1997 |
| WO 0107364 | A | | 01-02-2001 | AT | 250007 | T | 15-10-2003 |
| | | | | AU | 6436300 | A | 13-02-2001 |
| | | | | CZ | 20020310 | A3 | 15-01-2003 |
| | | | | DE | 60005367 | D1 | 23-10-2003 |
| | | | | DE | 60005367 | T2 | 24-06-2004 |
| | | | | EP | 1200346 | A1 | 02-05-2002 |
| | | | | ES | 2213031 | T3 | 16-08-2004 |
| | | | | HU | 0204138 | A2 | 28-03-2003 |
| | | | | PL | 354091 | A1 | 29-12-2003 |
| | | | | TR | 200200235 | T2 | 22-07-2002 |
| | | | | WO | 0107364 | A1 | 01-02-2001 |
| | | | | ZA | 200200537 | A | 25-06-2003 |
| WO 9854090 | A | | 03-12-1998 | AT | 244683 | T | 15-07-2003 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE
RELATIF A LA DEMANDE DE BREVET EUROPEEN NO.**

EP 16 16 3156

16-06-2016

| Document brevet cité au rapport de recherche | Date de publication | Membre(s) de la famille de brevet(s) | Date de publication |
|---|---|---|---|
| | | AU 743439 B2 | 24-01-2002 |
| | | BR 9804926 A | 08-09-1999 |
| | | CA 2261618 A1 | 03-12-1998 |
| | | CN 1230936 A | 06-10-1999 |
| | | DE 69816233 D1 | 14-08-2003 |
| | | DE 69816233 T2 | 03-06-2004 |
| | | EP 0917519 A1 | 26-05-1999 |
| | | ES 2198055 T3 | 16-01-2004 |
| | | FR 2763581 A1 | 27-11-1998 |
| | | ID 21812 A | 29-07-1999 |
| | | JP 3325029 B2 | 17-09-2002 |
| | | JP H11513976 A | 30-11-1999 |
| | | KR 20000029476 A | 25-05-2000 |
| | | PL 331327 A1 | 05-07-1999 |
| | | PT 917519 E | 28-11-2003 |
| | | RU 2170212 C2 | 10-07-2001 |
| | | TR 9900182 T1 | 21-07-1999 |
| | | US 6468493 B1 | 22-10-2002 |
| | | US 2003118500 A1 | 26-06-2003 |
| | | US 2005135985 A1 | 23-06-2005 |
| | | US 2007181036 A1 | 09-08-2007 |
| | | US 2011287257 A1 | 24-11-2011 |
| | | US 2015010757 A1 | 08-01-2015 |
| | | WO 9854090 A1 | 03-12-1998 |
| EP 0520862 A | 30-12-1992 | AT 192121 T | 15-05-2000 |
| | | AU 647282 B2 | 17-03-1994 |
| | | BR 9202439 A | 09-02-1993 |
| | | CA 2072399 A1 | 27-12-1992 |
| | | CN 1069244 A | 24-02-1993 |
| | | DE 69230959 D1 | 31-05-2000 |
| | | DE 69230959 T2 | 05-10-2000 |
| | | DK 0520862 T3 | 07-08-2000 |
| | | EP 0520862 A1 | 30-12-1992 |
| | | ES 2148167 T3 | 16-10-2000 |
| | | FI 922955 A | 27-12-1992 |
| | | FR 2678259 A1 | 31-12-1992 |
| | | GR 3033758 T3 | 31-10-2000 |
| | | IE 922066 A1 | 30-12-1992 |
| | | JP 3411819 B2 | 03-06-2003 |
| | | JP H0674127 B2 | 21-09-1994 |
| | | JP H05201719 A | 10-08-1993 |
| | | JP H11130420 A | 18-05-1999 |
| | | PT 520862 E | 31-08-2000 |
| | | TW 270918 B | 21-02-1996 |
| | | US 5403570 A | 04-04-1995 |

EPO FORM P0460

**ANNEXE AU RAPPORT DE RECHERCHE EUROPEENNE
RELATIF A LA DEMANDE DE BREVET EUROPEEN NO.**

EP 16 16 3156

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-06-2016

| Document brevet cité au rapport de recherche | Date de publication | Membre(s) de la famille de brevet(s) | Date de publication |
|---|---|---|---|
| | | US 5547502 A | 20-08-1996 |
| | | US 5587416 A | 24-12-1996 |
| | | ZA 9204651 A | 31-03-1993 |

Pour tout renseignement concernant cette annexe : voir Journal Officiel de l'Office européen des brevets, No.12/82

EPO FORM P0460

## RÉFÉRENCES CITÉES DANS LA DESCRIPTION

**Documents brevets cités dans la description**

- WO 9928376 A
- WO 9928380 A
- WO 0073372 A
- WO 0073373 A

**Littérature non-brevet citée dans la description**

- **BRUNAUER ; EMMET ; TELLER.** *The journal of the American Chemical Society,* Février 1938, vol. 60, 309
- **G. W. SEARS.** Determination of specific surface area of colloidal silica by titration with sodium hydroxyde. *Analytical Chemistry,* 12 Décembre 1956, vol. 28